# EUROPEAN PATENT SPECIFICATION

(11) **EP 1 556 071 B1**
(45) Date of publication and mention of the grant of the patent: **19.01.2011**
(21) Application number: 03779310.6
(22) Date of filing: 24.10.2003
(51) Int. Cl.: C07K 16/22, G01N 33/50, A61K 39/395, A61K 38/00

(54) **METHODS TO PREVENT TUMOR RECURRENCE BY BLOCKADE OF TGF-BETA**
VERFAHREN ZUR PRÄVENTION VON TUMORREZIDIVEN DURCH BLOCKADE VON TGF-BETA
METHODES POUVANT EMPECHER LA RECURRENCE D'UNE TUMEUR PAR BLOCAGE DE TGF-BETA

(30) Priority: 25.10.2002 US 421286 P
(43) Date of publication of application: 27.07.2005
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US)
(72) Inventor: BERZOFSKY, Jay, A., Bethesda, MD 20814-1107 (US); TERABE, Masaki, Bethesda, MD 20814 (US); MATSUI, So, Tsukuba-Shi, Ibaraki, 300-0051 (JP)
(74) Representative: Walton, Seán Malcolm
(86) International application number: PCT/US2003/034023
(87) International publication number: WO 2004/037209

(56) References cited:
- US-A- 6 090 383
- BRISTOW ROBET E ET AL: "Altered expression of transforming growth factor-beta ligands and receptors in primary and recurrent ovarian carcinoma" CANCER, vol. 85, no. 3, 1 February 1999 (1999-02-01), pages 658-668, XP002529307 ISSN: 0008-543X
- SAITO H ET AL: "An elevated serum level of transforming growth factor-beta 1 (TGF-beta 1) significantly correlated with lymph node metastatis and poor prognosis in patients with gastric carcinoma" ANTICANCER RESEARCH, HELENIC ANTICANCER INSTITUTE, ATHENS, GREECE, vol. 20, no. 6B, 1 November 2000 (2000-11-01), pages 4489-4493, XP002962704 ISSN: 0250-7005
- MAO X.W. ET AL: 'Immunotherapy with low-dose interleukin-2 and anti-transforming growth factor-beta antibody in a murine tumor model' CANCER BIOTHERAPY vol. 9, no. 4, 1994, pages 317 - 327, XP000886027

## Description

### FIELD

The present disclosure is related to methods of preventing tumor recurrence. More specifically, the disclosure relates to methods of blocking transforming growth factor (TGF)-β signaling in order to inhibit the immunosuppressive effects of TGF-β thereby preventing the recurrence of a tumor.

### BACKGROUND

The transforming growth factor (TGF)-β superfamily comprises a large group of multifunctional polypeptide growth and differentiation factors whose family members are grouped into three classes based on a variety of structural and functional criteria (U.S. Patent No. 6,046,165). The three classes include: 1) TGF-β isoforms; 2) activins; and 3) bone morphogenic proteins. More distantly related members of this protein family include murine *nodal* gene products, *Drosophila* decapentaplegic complex gene products, and Vg1 from *Xenopus*.

TGF-β is a homodimeric protein of which there are at least five isoforms. In general, TGF-β family proteins are homodimers, wherein each functional protein complex includes two identical, associated monomer subunits. The crystal structure of the TGF-β1 homodimer is known (Hinck et al., Biochem., 35:8517, 1996; Qian et al., J. Biol. Chem, 271:30656, 1996). TOP-β is a very compact protein, having four intramolecular disulfide bridges within each subunit, as well as one intermolecular disulfide bridge.

Each monomer of the protein is synthesized as a large (~55 kDa) precursor molecule with a long (about 278 residue) N-terminal pro-region and a much shorter (112 residue, 12.5 kDa) C-terminal active domain (the mature region). During the maturation process, two precursor molecules associate with each other; the pro-region is important for proper folding of and proper association between the two active domain monomers. The pro-region of each monomer is proteolytically cleaved from the associated active domain; in most instances however, the pro-region remains associated with the mature TGF-β fragment. For a discussion of TGF-β synthesis, see Khalil, Micro. Infect., 1:1255, 1999.

The members of the TGF-β family act by stimulating the formation of specific heteromeric complexes containing type I and type II transmembrane serine/threonine kinase receptors. The type II receptor binds ligand and phosphorylates the type I receptor in its glycine- and serine-rich domain. This series of molecular events transmits a signal to downstream molecules, the Smads, ultimately influencing protein expression and cell function (see, for example Moustakas et al., J Cell Science 114:4359, 2001; Massague and Wotton, EMBO J. 19:1745, 2000).

TGF-βs and their receptors are expressed in essentially all tissues, and have been found to be important in many cellular processes since TGF-β has been shown to play a role in cell growth and differentiation, immunosupression, inflammation, and the expression of extracellular matrix proteins. For example, TGF-β inhibits the growth of many cell types, including epithelial cells, but it also has been shown to stimulate the proliferation of various types of mesenchymal cells. By way of another example, in animal models TGF-β has been shown to attenuate the symptoms associated with various diseases and disorders, including rheumatoid arthritis, multiple sclerosis, wound healing, bronchial asthma, and inflammatory bowel disease, and has been used in the clinical setting to enhance wound healing. TGF-β also has many immunoregulatory functions, including modulation of T cell proliferation, apoptosis, activation and differentiation.

TGF-βs are expressed in large amounts in many tumors and are believed to have two important roles in cancer as discussed, for instance, in U.S. Patent No. 6,046,165. TGF-β is generally growth inhibitory. However, TGF-β is also highly immunosuppressive, thus it has been proposed that TGF-β is involved in tumor "escape." Tumor cells that are no longer responsive to the growth inhibitory effects of TGF-β up-regulate the expression of the cytokine to protect themselves from the immune system and thereby escape immunosurveillance (Mule et al., Cancer Immunol Immunother 26:95, 1988; Gorelik and Flavell, Nature Medicine 7:1118, 2001).

### SUMMARY OF THE DISCLOSURE

The disclosure provides antibodies for use in methods of inhibiting tumor recurrence in a subject by administering a therapeutically effective amount of the antibody to the subject. The antibody neutralizes the activity of TGF-β, which blocks the immunosuppressive effects of TGF-β and prevents recurrence of the tumor. The antibody binds TGF-β. .

Described herein are methods for enhancing an immune response in a subject to inhibit tumor recurrence. Examples of such methods include administering to a subject a therapeutically effective amount of an agent which blocks the TGF-β signaling pathway in an immune cell that expresses a TGF-β receptor. Methods for enhancing the activity of an immune cell to inhibit tumor recurrence are also described. Examples of such methods include contacting an immune cell that expresses a TGF-β receptor with an agent which blocks a TGF-β signaling pathway. Also disclosed are methods of screening for an agent that inhibits or measurably reduces the recurrence of a tumor, or that enhances an immune response.

The foregoing and other features and advantages will become more apparent from the following detailed description of several embodiments, which proceeds with reference to the accompanying figures.

### BRIEF DESCRIPTION OF THE FIGURES

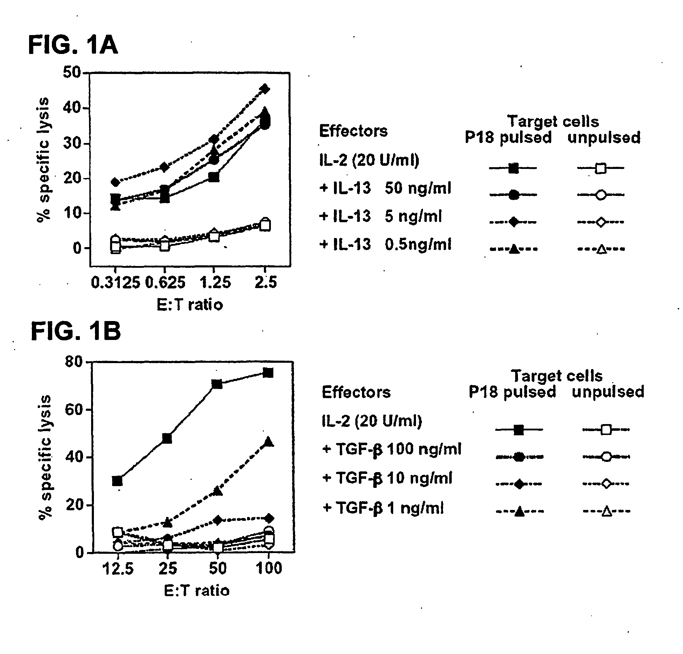

**FIG.1** is a pair of graphs illustrating that TGF-β1, but not interleukin (IL)-13, can directly suppress cytotoxic T lymphocyte (CTL) induction *in vitro.* Spleen cells from mice previously immunized with vPE16 (4 × 10⁶) were stimulated *in vitro* with 1 × 10⁶ P18-pulsed spleen cells for 6 days. During *in vitro* stimulation, the cells were maintained either (FIG. 1A) in the presence of IL-2 alone (squares) or in the presence of IL-2 plus 50 ng/ml (circles), 5 ng/ml (diamonds) or 0.5 ng/ml (triangles) ofIL-13, or (FIG.1B) in the presence of IL-2 alone (squares) or in the presence of 100 ng/ml (circles), 10 ng/ml (diamonds) or 1 ng/ml (triangles) of TGF-β. The cultured cells were used for CTL assay at the E:T ratios shown. Targets were 18Neo fibroblasts either without peptide (open symbols) or pulsed with 1 µM P18-IIIB peptide (closed symbols). The data shown are representative of three independent experiments.

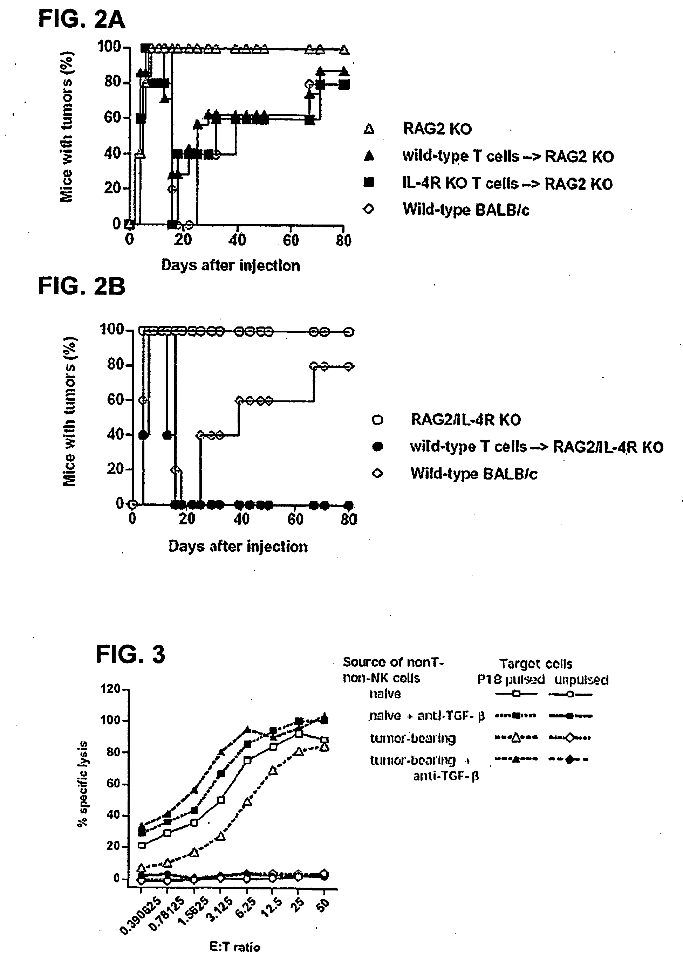

**FIG. 2** is a pair of graphs illustrating that non-T and non-B cells are IL-13-responding cells that down-regulate tumor immunosurveillance. RAG2 knockout (KO) and RAG2/IL-4Rα KO mice were injected intravenously with 5 x 10⁷ purified T cells from spleens of wild-type or IL-4Rα KO mice. One week later, the mice were injected subcutaneously with 1 x 10⁶ 15-12RM tumor cells. Five mice were used for each group. The result shown is representative of three experiments.
**FIG. 3** is a graph illustrating that TGF-β1 directly suppresses CTL induction *in vitro*. On day 3 after 15-12RM injection, non-T-non-natural killer (NK) cells were prepared from spleen cells of tumor-bearing (triangles and diamonds) and naive BALB/c mice (squares and circles) by negatively depleting CD4⁺, CD8⁺ and DX5⁺ cells, and pulsed with 1 µM of P18 peptide. Splenic T cells from vPE 16-immunized mice were stimulated *in vitro* in the presence of these non-T-non-NK cells for a week. In addition to IL-2 added to all cultures, 50 µg/ml of anti-TGF-β was added (closed symbols) or omitted (open symbols) during *in vitro* stimulation. The cultured cells were harvested and used for CTL assay. Targets were 18Neo fibroblasts pulsed either with P18 peptide (squares and triangles) or without peptide (circles and diamonds). A similar result was obtained in another independent experiment.

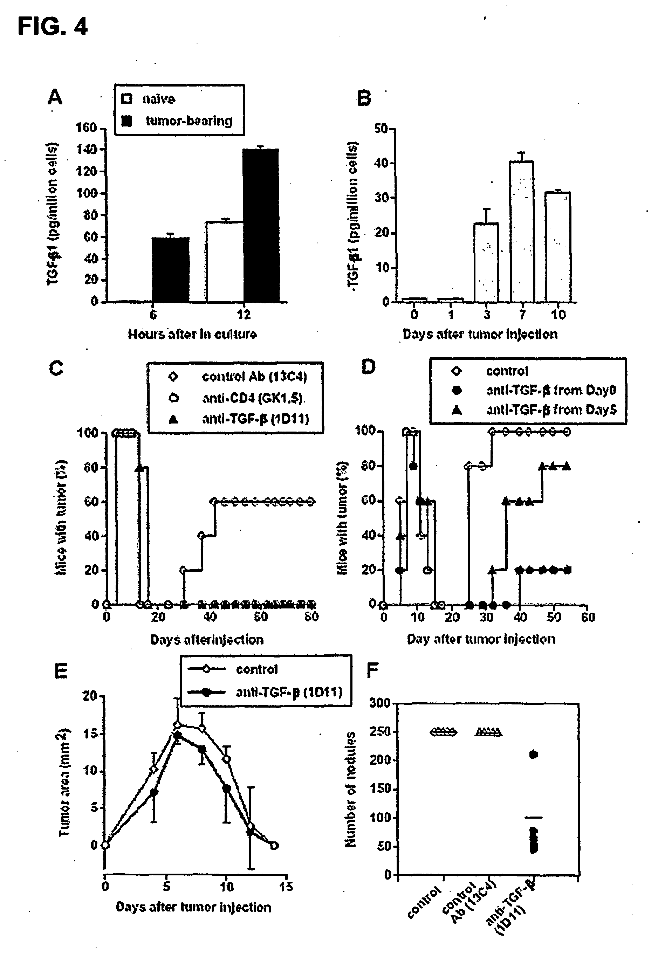

**FIG. 4** is a series of graphs showing that TGF-β1 production was increased in non-lymphoid spleen cells (non-T-non-B-non-NK cells) obtained from 15-12RM-injected BALB/c mice and that the anti-TGF-β antibody prevents tumor recurrence. FIG. 4A shows that on day 3 after 15-12RM injection, freshly isolated non-lymphoid cells from naïve BALB/c (white bar) and 15-12RM tumor-injected BALB/c mice (filled bar) were examined for TGF-β1 production *ex vivo*. Error bars not shown are too small to be seen on this scale. This experiment is representative of 10 experiments with similar results. FIG. 4B shows freshly isolated non-lymphoid cells prepared from 15-12RM-injected mice at different time points after tumor injection that were examined for TGF-β1 production *ex vivo* as described in FIG. 4A. FIG. 4C shows anti-CD4-treated mice (circles), and-TGF-β-treated mice (triangles) or control isotype matched antibody-treated (diamonds) BALB/c mice (5/group) that were injected with 1 × 10⁷ 15-12RM cells subcutaneously. *p*<0.05 with Log-Rank test between the control group and the anti-TGF-β group. This experiment is representative of three experiments with similar results. FIG. 4D shows that after subcutaneous inoculation of 15-12RM cells, the mice (5/group) were treated with 100 µg of anti-TGF-β antibody from day 0 (filled circle) or day 5 (filled triangle), or control isotype-matched antibody (diamonds) every other day for 10 days. FIG. 4E shows the size of primary tumors in 15-12RM-injected BALB/c mice treated with 100 µg of anti-TGF-β monoclonal antibody (circles) intraperitonealy every other day for 10 days or without any antibody treatment (diamonds). The vertical axis shows tumor area measured as the product of these two dimensions. This experiment is representative of 4 independent experiments. In FIG. 4F, BALB/c mice (5/group) injected with 2 × 10⁵ CT26 cells treated with 0.1 mg of anti-TGF-β monoclonal antibody or isotype matched control monoclonal antibody. The mean number of nodules is indicated as horizontal bars. *p*<0.0001 with one way ANOVA test for the anti-TGF-β group compared to control and control mAb groups. Similar results were obtained in a duplicate experiment.

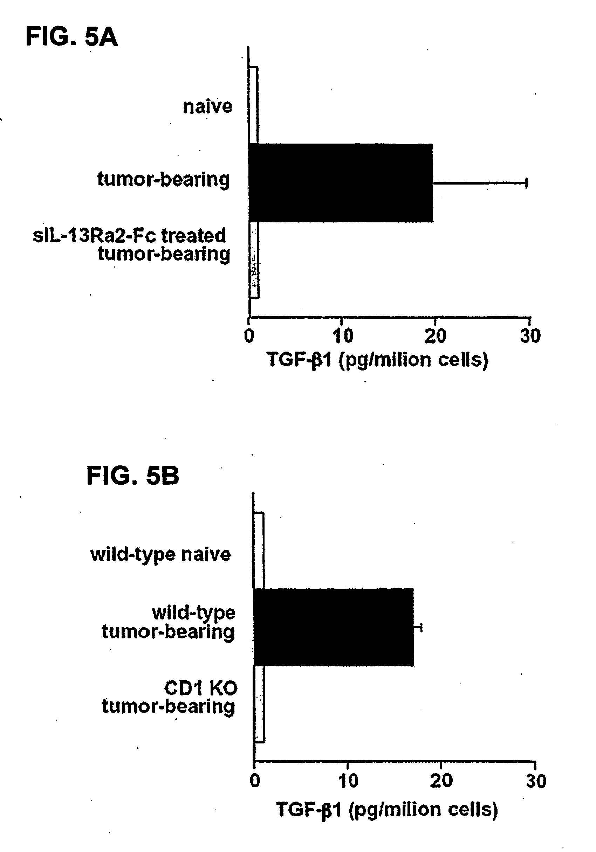

**FIG. 5** is a pair of graphs illustrating that *in vivo* TGF-β1 production is down-regulated in IL-13 inhibitor treated wild-type mice or CD1 KO mice injected with 15-12RM tumor cells. FIG. 5A shows that, on day three after 15-12RM tumor injection, freshly isolated non-lymphoid cells from naive BALB/c (white bar), 15-12RM tumor-injected BALB/c mice (black bar), and 15-12RM tumor-injected BALB/c mice treated with IL-13 inhibitor (gray bar) were examined for TGF-β1 production *ex vivo.* IL-13 inhibitor (sIL-13Rα2-Fc, 0.2 mg) was administrated intraperitonealy on days 0, 1, 2 after tumor injection. FIG. 5B shows that naive wild-type BALB/c, 15-12RM tumor-injected wild-type BALB/c and tumor-injected CD1 KO BALB/c mice were used as a source of non-lymphoid cells on day three of tumor injection. Two hundred thousand cells were cultured *in vitro* without particular stimulation. The culture supernatant was collected at the indicated time and the concentration of TGF-β1 was determined by ELISA (enzyme linked immunosorbent assay). Each value shows the average ± SD of triplicate assay. Error bars not shown are too small to be seen on this scale. Similar results were obtained in three independent experiments.

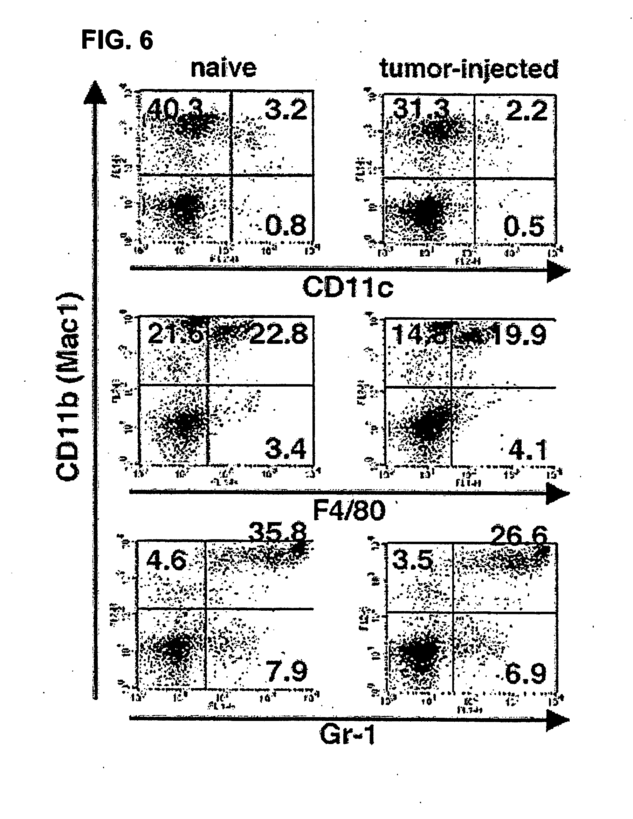

**FIG. 6** is a series of fluorescence activated cell sorting (FACS) plots, showing flow cytometric staining of splenic non-lymphoid cells from naive and 15-12RM tumor-injected mice. Non-lymphoid spleen cells were obtained from naive and 15-12RM-injected mice (day 3) by depleteing CD4⁺, CD8⁺, B220⁺, and DX5⁺ cells with magnetic beads. The cells were stained with anti-Gr-1, anti-CD11c, antiF4/80, and anti-CD11b antibodies for 30 minutes after blocking CD16/CD32 for 15 minutes. The cells were washed once and fixed with Cytofix/Cytoperm (Pharmingen), and washed with Perm/Wash buffer. After washing, the cells were analyzed by FACScan or FACS Caliber by using CELLQuest software (BD Biosciences). This experiment is representative of ten experiments with similar results.

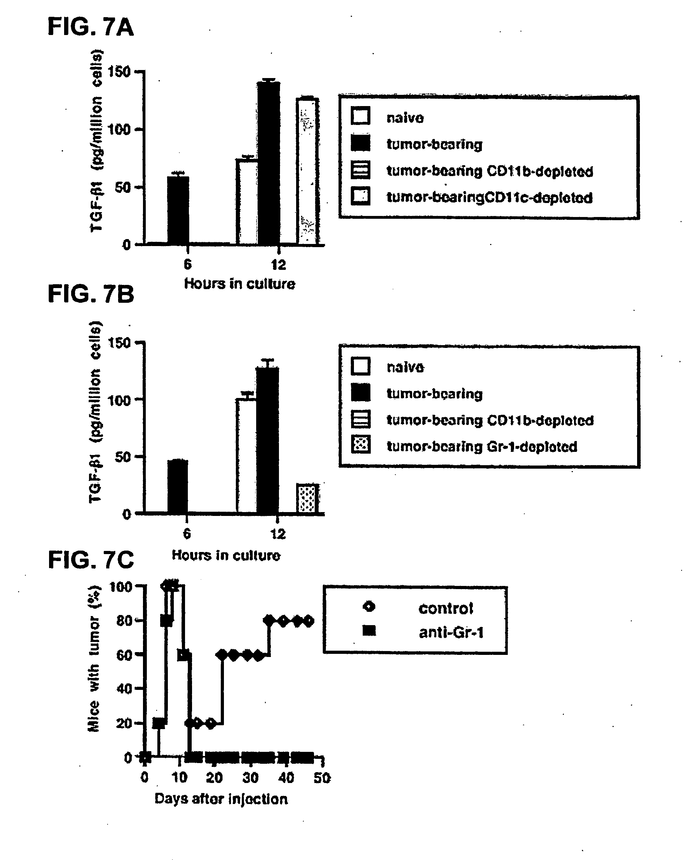

**FIG. 7** is a series of graphs illustrating that CD11b⁺ cells and Gr-1⁺ cells are the major source of TGF-β1 and are necessary for the down-regulation of tumor immunosurveillance. FIG. 7A shows the amount of TGF-β1 produced from CD11b⁺ (hatched bars) or CD11c⁺ (gray bar) depleted cells following purification of the cells from 15-12RM tumor-injected mice. FIG. 7B shows the amount of TGF-β1 produced from CD11b⁺ (hatched bars) or Gr-1⁺ (dotted bars) depleted cells following purification of the cells from 15-12RM tumor-injected mice. Error bars not shown are too small to be seen on this scale. This experiment is representative of three experiments with similar results. FIG. 7C is a graph showing the percent of anti-Gr-1-treated mice (squares) or control BALB/c mice (diamonds) with tumors. Mice were injected with 1 × 10⁷ 15-12RM cells subcutaneously. The anti-Gr-1 antibody (1 µg) was intraperitonealy inoculated on day 5, 6, 10, 15 and 20 after 15-12RM injection. Five mice were used for each group. *p*<0.05 with Log-Rank test between the control group and the anti-Gr-1-treated group. This experiment is representative of three experiments with similar results.

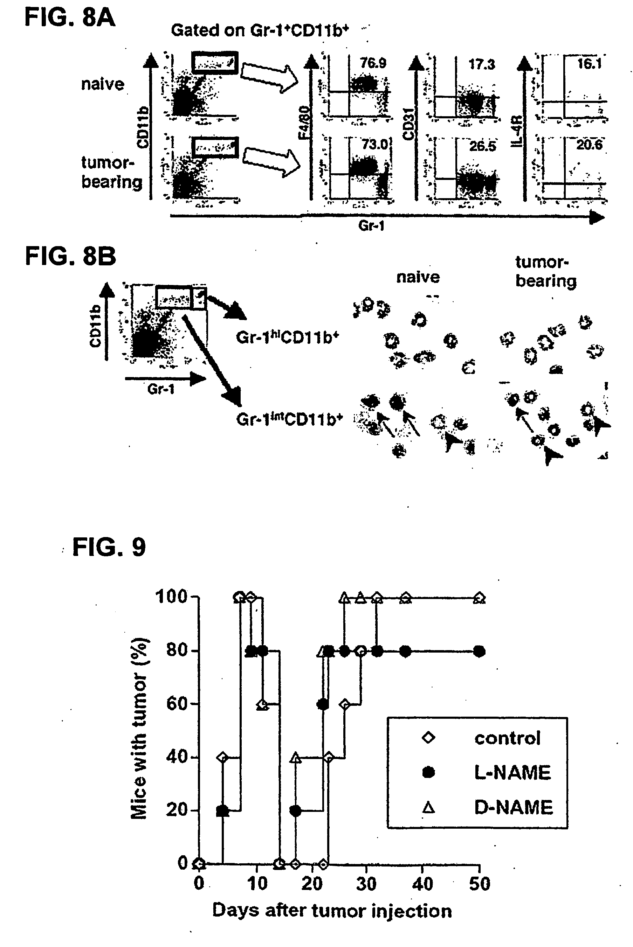

**FIG. 8** is a series of images illustrating the characterization of Gr-1⁺CD11b⁺ cells. On day 3 after 15-12RM injection, single-cell suspensions of spleen cells from tumor-bearing and naïve BALB/c mice were prepared. FIG. 8A shows the percentage of the cells positive for each cell surface marker among the Gr-1⁺CD11b⁺ cells. FIG. 8B shows non-lymphoid cells stained with FITC-conjugated anti-Gr-1, Per-CP conjugated anti-CD11b antibodies. The cells were preparatively sorted by flow cytometry to select Gr-1^{hi}CD11b+ (upper panels) and Gr-1^{int}CD11b⁺ populations (lower panels). The sorted cells were collected by cytospin on glass slides, dried over night, and stained with Wright-Giemsa stain. Pictures were taken under a microscope at 20x magnification. Arrows indicate monocytes and arrowheads indicate immature granulocytes with "band" morphology.
**FIG. 9** is a graph illustrating that *in vivo* blockade of nitric oxide production did not alter tumor growth. Mice received 0.2 mg of L-NAME (*N*-nitro-L-arginine-methyl ester), which is believed to inhibit iNOS (inducible nitric oxide synthase) *in vivo*, or D-NAME (*N*-nitro-D-arginine-methyl ester) every day for two weeks after tumor injection. The number of mice with tumors was measured up to day 50 after tumor injection. Similar results were obtained in an independent experiment.

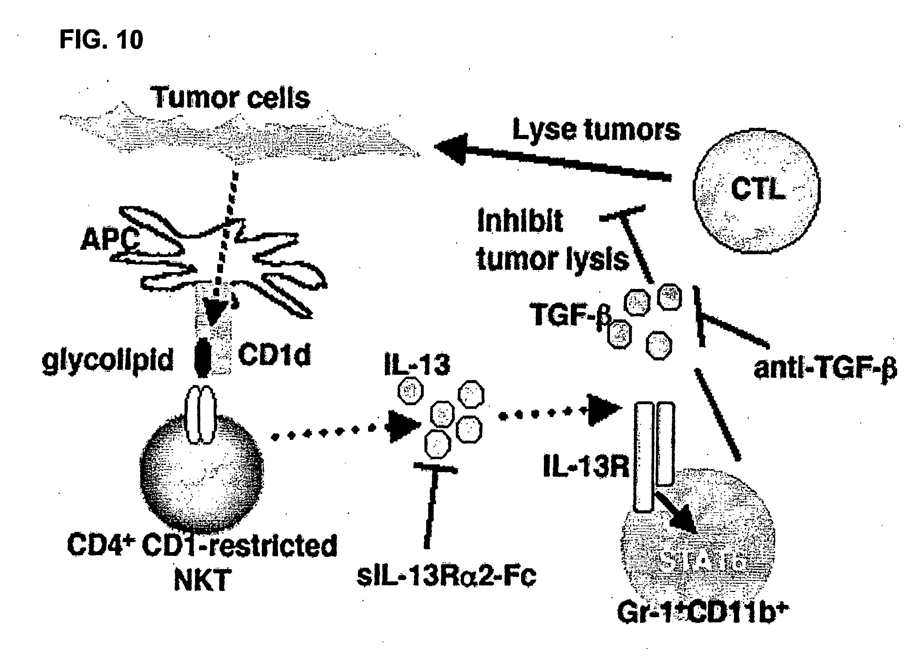

**FIG.10****.** is a schematic drawing of a proposed model of negative immunoregulatory circuit of CTL-mediated tumor immunosurveillance mediated by TGF-β, myeloid cells, IL-13 and CD4⁺ CD1d-restricted T cells (possibly NKT cells). Tumor antigen (glycolipid) presented by antigen presenting cells via the CD1d molecule is recognized by and activates CD4⁺ CD1d-restricted NKT cells. The activated CD4⁺ CD1d-restricted NKT cell produces IL-13 which acts on Gr-1⁺CD11b⁺ myeloid cells which express the IL-13 receptor. The GR-1⁺CD11b⁺ myeloid cell produces TGF-β to suppress CD8⁺ cytotoxic T cells (CTL) that can kill tumor cells, thereby down-regulating tumor immunosurveillance. This pathway is able to be blocked by IL-13 inhibitor and anti-TGF-β antibody.

### DETAILED DESCRIPTION

### L Abbreviations

- Cr: chromium
- CTL: cytotoxic T lymphocyte
- CTM: complete T cell medium
- ELISA: enzyme linked immunosorbent assay
- ELISPOT: enzyme-linked immunospot
- FACS: fluorescence activated cell sorting
- GM-CSF: granulocyte-macrophage colony stimulating factor
- IL: interleukin
- IFN: interferon
- iNOS: inducible nitric oxide synthase
- KO: knockout
- MHC: major histocompatibility complex
- NK: natural killer
- NO: nitric oxide
- TGF: transforming growth factor
- TNF: tumor necrosis factor

### II. Terms

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of the invention, the following explanations of specific terms are provided:

**Activity of a TGF-β receptor expressing immune cell:** A biological activity of a cell that expresses a TGF-β receptor. The biological activity of such a cell can include target cell lysis, cell proliferation, cytokine production, inhibition of tumor growth, or inhibition of tumor recurrence. A change in activity of a cell that expresses a TGF-β receptor, such as a reduction in target cell lysis, cytokine production, or inhibition of tumor recurrence, can result from a blockade of TGF-β signaling. The ability to measure the activity of a cell can be performed by any method know to one of skill in the art. For example, the ability to lyse a target cell can be measured by a chromium (Cr) release assay, as disclosed herein. In another example, the ability to produce cytokines can be measured by western blot or northern analysis. In yet another example, the ability to inhibit tumor recurrence can be measured by the number of mice with tumors following treatment (for example, following administration of an anti-TGF-β antibody) versus control mice.

**Agent:** Any substance, including, but not limited to, an antibody, chemical compound, small molecule, peptide mimetic, peptide or protein. The agent can be produced by a subject's body. In one embodiment, an agent blocks the immunosuppressive effects of TGF-β. The agent inhibits or reduces a recurrence of a tumor. In another embodiment, an agent prevents a recurrence of a tumor. The agent neutralizes an activity of a TGF-β.

**Animal:** Living multi-cellular vertebrate organisms, a category that includes, for example, mammals and birds. The term mammal includes both human and non-human mammals. Similarly, the term "subject" includes both human and veterinary subjects.

**Antibody:** Immunoglobulin (Ig) molecules and immunologically active portions of Ig molecules, for instance, molecules that contain an antigen binding site which specifically binds (immunoreacts with) an antigen. In the embodiments the antigen is TGF-β. The TGF-β receptor and TGF-β downstream signaling molecules (for example, Smad2, Smad3, Smad4, Smad complex DNA-binding co-factors) are also described Monoclonal, polyclonal, and humanized immunoglobulins are encompassed by the disclosure. The disclosure also includes synthetic and genetically engineered variants of these immunoglobulins.

A naturally occurring antibody (for example, IgG) includes four polypeptide chains, two heavy (H) chains and two light (L) chains inter-connected by disulfide bonds. However, it has been shown that the antigen-binding function of an antibody can be performed by fragments of a naturally occurring antibody. Thus, these antigen-binding fragments are also intended to be designated by the term "antibody". Examples of binding fragments encompassed within the term antibody include (i) an Fab fragment consisting of the VL, VH, CL and CH1 domains; (ii) an Fd fragment consisting of the VH and CH1 domains; (iii) an Fv fragment consisting of the VL and VH domains of a single arm of an antibody, (iv) a dAb fragment (Ward et al., Nature 341:544, 1989) which consists of a VH domain; and (v) an F(ab')₂ fragment, a bivalent fragment comprising two Fab fragments linked by a disulfide bridge at the hinge region.

Furthermore, although the two domains of the Fv fragment are coded for by separate genes, a synthetic linker can be made that enables them to be made as a single protein chain (known as single chain Fv (scFv); Bird et al. Science 242:423, 1988; and Huston et al. Proc. Natal. Acad. Sci. 85:5879, 1988) by recombinant methods. Such single chain antibodies, as well as dsFv, a disulfide stabilized Fv (Bera et al. (1998) J. Mol. Biol. 281:475-483), and dimeric Fvs (diabodies), that are generated by pairing different polypeptide chains (Holliger et al. (1993) Proc. Natl. Acad. Sci. 90:6444-6448), are also included.

In one embodiment, antibody fragments for use in this disclosure are those which are capable of cross-linking their target antigen, for example, bivalent fragments such as F(ab')₂ fragments. Alternatively, an antibody fragment which does not itself cross-link its target antigen (for example, a Fab fragment) can be used in conjunction with a secondary antibody which serves to cross-link the antibody fragment, thereby cross-linking the target antigen. Antibodies can be fragmented using conventional techniques and the fragments screened for utility in the same manner as described for whole antibodies. An antibody is further intended to include humanized monoclonal molecules that specifically bind the target antigen.

"Specifically binds" refers to the ability of individual antibodies to specifically immunoreact with an antigen. This binding is a non-random binding reaction between an antibody molecule and the antigen. In the embodiments, the antigen is a TGF-β. Binding specificity is typically determined from the reference point of the ability of the antibody to differentially bind the antigen of interest and an unrelated antigen, and therefore distinguish between two different antigens, particularly where the two antigens have unique epitopes. An antibody that specifically binds to a particular epitope is referred to as a "specific antibody". In the embodiments the monoclonal antibody is obtained from hybridoma 1D11.16 (ATCC Accession No. HB 9849) binds TGF-β and therefore is specific.

**Antigen:** Any molecule that is specifically bound by an antibody. An antigen is also a substance that antagonizes or stimulates the immune system to produce antibodies, for example an antigen on the surface of an antigen-presenting cell. Antigens are often substances, such as allergens, bacteria or viruses, that invade the body.

In the embodiments the antigen is a TGF-β. The TGF-β receptor or a TGF-β downstream signaling molecules (for example, Smad2, Smad3, Smad4, Smad complex DNA-binding co-factors), are also described.

**Cytokines:** Proteins, made by cells, that mediate inflammatory and immune reactions. In one embodiment, a cytokine is a chemokine, a molecule that affects cell movement Cytokines include, but are not limited to, interleukins (for example, IL-4, IL-8, IL-10, IL-13), granulocyte-macrophage colony stimulating-factor (GM-CSF), neurokinin, tumor necrosis factors (TNFs) (for example, TNF-α, TNF-β), interferons (IFNs) (for example, IFN-α, IFN-β, IFN-γ) and TGF-βs (for example, TGP-β-1, TGF-β-2).

**Cytotoxic T lymphocyte (CTL):** A lymphocyte that is able to kill either self cells presenting foreign antigens, or abnormal self cells, including tumor cells, marked for destruction by the cellular immune system. CTLs can destroy cells infected with viruses, fungi, parasites, or certain bacteria. CTLs usually express the CD8 cell surface marker and recognize microbial peptides displayed by class I major histocompatibility complex (MHC) molecules. CTLs kill virus-infected cells, whereas antibodies generally target free-floating viruses in the blood. CTL killing of infected cells involves the release of cytoplasmic granules whose contents include membrane pore-forming proteins and enzyme. CTLs perform an immune surveillance function by recognizing and killing potentially malignant cells that express peptides that are derived from mutant cellular proteins or oncogenic viral proteins and are presented in association with class I MHC molecules. CTL-mediated tumor immunosurveillance is down-regulated by TGF-β as disclosed herein.

**CTL assay:** Activated CTLs generally kill any cells that display the specific peptide:MHC class I complex they recognize. CTL activity can be determined by using an assay that measures the ability of a CTL to kill a target cell (a cell expressing a specific peptide:MHC class I complex). The classical assay for CTL activity is the chromium release assay. Target cells expressing an antigen on their surface are labeled with a radioactive isotope of chromium (⁵¹Cr). CTLs of a subject are then mixed with the target cell and incubated for several hours. Lysis of antigen-expressing cells by CTLs releases ⁵¹Cr into the medium which can be detected and quantified. The ability of CTLs to cause antigen-specific lysis is calculated by comparing lysis (correlated with chromium release) of target cells expressing the antigen or control antigens in the presence or absence of effector cells, and is usually expressed as the percent antigen-specific lysis.

**Epitope tags:** Short peptides or sequences of amino acids to which an antibody can be generated. In some embodiments, peptide tags allow one to specifically identify and track a protein of interest, for example a tagged protein that has been added to a living organism or to cultured cells. Detection of the tagged protein can be achieved using a number of different techniques. Examples of such techniques include: immunohistochemistry, immunoprecipitation, flow cytometry, immunofluorescence microscopy, ELISA, immunoblotting ("western"), and affinity chromatography. Examples of useful epitope tags include FLAG, T7, HA (hemagglutinin) and myc.

**Immune cell:** Any cell involved in a host defense mechanism. These include, for example, T cells, B cells, natural killer (NK) cells, NKT cells, neutrophils, mast cells, macrophages, antigen-presenting cells, basophils, eosinophils, and neutrophils.

**Immune response:** A collective and coordinated response to the introduction of a foreign (for example, non-self) substance in a subject, which response is mediated by the cells and molecules of the immune system. One example of an immune response is CTL-mediated tumor immunosurveillance. Another example of an immune response is one that is specific for a particular antigen (an "antigen-specific response"), such as a tumor-specific antigen. Yet another example of an immune response is one that is stimulated by the presence of a cytokine.

**Immunosuppression:** Inhibition of one or more components of the adaptive or innate immune system as a result of an underlying disease, or intentionally induced by drugs for the purpose of preventing or treating graft rejection or autoimmune disease (in Cellular and Molecular Immunology, fourth edition, WB Saunders Co., 2000).

**Immunosuppressive agent:** An agent that has an inhibitory effect on at least one function of the immune response and that causes immunosuppression. One example of an immunosuppressive agent is TGF-β. An immunosuppressive agent can prevent the immune system from reacting to foreign (non-self) substances and fighting disease, such as a tumor or other abnormal growth.

TGF-β is highly immunosuppressive as illustrated by the fact that CD8⁺ CTL-meditated tumor immunosurveillance is down-regulated by TGF-β. It has been proposed that TGF-β is involved in tumor "escape." Tumor cells that are no longer responsive to the growth-inhibitory effects of TGF-β up-regulate the expression of TGF-β to protect themselves from the immune system and thereby escape immunosurveillance (Mule et al., Cancer Immunol Immunother 26:95, 1988; Gorelik and Flavell, Nature Medicine 7:1118, 2001).

The mechanisms of down-regulation of tumor immunosurveillance and immunosuppression by TGF-β can be studied, for instance, using a mouse tumor model in which tumors shows a "growth-regression-recurrence" pattern following tumor inoculation in the mouse. One such system is described in Example 5.

**Immunosurveillance:** Function of the immune system to recognize and destroy cells that express a foreign antigen (for example, tumor or microbial antigens). In one embodiment, immunosurveillance is the function of T lymphocytes to recognize and destroy transformed cells before they grow into tumors, and to kill tumors after they are formed. One specific, non-limiting example of immunosurveillance is CD8⁺ CTL-mediated tumor immunosurveillance.

**Isolated:** An "isolated" biological component (such as a nucleic acid molecule, protein or organelle) has been substantially separated or purified away from other biological components in the cell of the organism in which the component naturally occurs, for instance, other chromosomal and extra-chromosomal DNA and RNA, proteins and organelles. Nucleic acids and proteins that have been "isolated" include nucleic acids and proteins purified by standard purification methods. The term also embraces nucleic acids and proteins prepared by recombinant expression in a host cell, as well as chemically synthesized biopolymers. The terms "isolated" does not require absolute isolation. Similarly, the term "substantially separated" does not require absolute separation.

**Lymphocytes:** A type of white blood cell that is involved in the immune response of the body. There are two main classes of lymphocytes: B-cells and T-cells. A third class of lymphocytes is Natural Killer (NK) cells. Cytotoxic T lymphocytes (CTL) and NKT cells are types of T cells.

**Mammal:** This term includes both human and non-human mammals. Similarly, the term "subject" includes both human and veterinary subjects.

**Metastasis:** The spread of a tumor from one part of the body to another. Tumors formed from cells that have spread are called "secondary tumors" and contain cells that are like those in the original (primary) tumor. Metastasis is caused by at least a single tumor cell that is derived from an original tumor and that circulates or migrates to a different site from the original tumor. Metastasis requires the establishment of a new blood supply at the new tumor site.

**Natural Killer (NK) cells:** A type of lymphocyte (neither a T cell nor a B cell) that does not express the CD3 cell surface marker and does not use a conventional T cell receptor or B cell receptor to recognize its target. NK cells have activating or inhibitory receptors that detect the presence or absence ofMHC molecules on target cells but, unlike T cell receptors, these are not antigen specific or MHC restricted.

NK cells provide part of the innate immune defense against virus-infected cells and cancer cells that is nonspecific. They do not have memory and are not induced by immunization with specific antigen. NK cells can mediate antibody-dependent cellular cytotoxicity (ADCC) through their Fc receptors. In the mouse, they have been identified by a surface marker called NK1.1,but are negative for the T cell markers CD3, CD4, and CD8. Subjects with immunodeficiencies, such as those caused by HIV infection, often have a decrease in "natural" killer cell activity.

**Neutralize:** Descriptive of an agent that can inhibit the activity of a molecule. Examples of a neutralizable molecule include TGF-β,the TGF-β receptor, or a TGF-β downstream signaling molecule. In one embodiment, neutralizing TGF-β inhibits the TGF-β signaling pathway, thereby inhibiting the immunosuppressive effects of TGF-β.Agents are disclosed herein to neutralize an activity of a molecule, for instance by any measure amount The term "neutralize" does not require absolute neutralization. Similarly, the term "inhibits" does not require absolute inhibition.

By way of example, an agent can neutralize a molecule by specifically binding it, thereby preventing the molecule from performing its function or one of its functions. In one embodiment, the neutralizing agent prevents a molecule from interacting with other molecules, for example by preventing TGF-β from interacting with the TGF-β receptor, thereby neutralizing an activity of TGF-β.One specific, non-limiting example of a neutralizing agent is the 1D11.16 anti-TGF-β monoclonal antibody.

**NKT cells:** T cells that express the CD3 cell surface marker and have a conventional type of alpha-beta T cell receptor, but the repertoire of the alpha-beta T cell receptor is limited, so that most NKT cells recognize a glycolipid antigen presented by the non-classical class I MHC molecule CD1d. CD1d molecules are MHC (major histocompatibility complex) class I-like molecules that present glycolipids, rather than peptides, to T lymphocytes. NKT cells use a limited repertoire ofT cell receptors, especially the V-alpha 14/ V-beta 8 pair in the mouse and the V-alpha 24 in the human. They have the ability to kill target cells, but one of their major functions is to secrete cytokines very early in an immune response. They all express CD3, and some express CD4, whereas some are CD4/CD8 double negative. They were originally described as NKT cells in the mouse because they express the NK1.1 marker, like NK cells, but that is their only similarity with NK cells. They are now more commonly defined as T cells that are CD1d restricted.

**Nucleotide:** This term includes, but is not necessarily limited to, a monomer that includes a base linked to a sugar, such as a pyrimidine, purine or synthetic analogs thereof, or a base linked to an amino acid, as in a peptide nucleic acid (PNA). A nucleotide is one monomer in a polynucleotide. The term also includes other art-obvious modifications of such molecules that can form part of a polynucleotide. A nucleotide sequence refers to the sequence of bases in a polynucleotide.

**Parenteral:** Administered outside of the intestine, for example, not via the alimentary tract. Generally, parenteral formulations are those that will be administered through any possible mode except ingestion. This term especially refers to injections, whether administered intravenously, intrathecally, intramuscularly, intraperitoneally, or subcutaneously, and various surface applications including intranasal, intradermal, and topical application, for instance.

**Peptide:** Any compound containing two or more amino-acid residues joined by amide bonds, formed from the carboxyl group of one residue and the amino group of the next. The broad term "peptide" includes oligopeptides, polypeptides, and proteins.

**Pharmaceutically acceptable carriers:** The pharmaceutically acceptable carriers useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of the fusion proteins herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Primary tumor:** The original tumor. A tumor at the original site (as opposed to a metastatic tumor).

**Protein:** A biological molecule expressed by an encoding nucleic acid molecule (for example, a gene) and comprised of amino acids. Proteins are a subset of the broader molecular class "peptide."

**Purified:** The term purified does not require absolute purity; rather, it is intended as a relative term Thus, for example, a "purified" protein preparation is one in which the protein is more enriched than the protein is in its generative environment, for instance within a cell or in a biochemical reaction chamber. Preferably, a preparation of protein is purified such that the protein represents at least 50%, at least 60%, at least 70%, at least 75%, at least 80%, at least 85%, at least 90%, at least 95%, or at least 99% of the total protein content of the preparation.

**Recombinant nucleotide:** A recombinant nucleotide is one that has a sequence that is not naturally occurring or has a sequence that is made by an artificial combination of two otherwise separated segments of nucleotide sequence. This artificial combination can be accomplished by chemical synthesis or, more commonly, by the artificial manipulation of isolated segments of nucleic acids, for example, by genetic engineering techniques. Similarly, a recombinant protein is one encoded for by a recombinant nucleotide.

**Sequence identity:** The similarity between two nucleic acid sequences, or two amino acid sequences, is expressed in terms of the similarity between the sequences, otherwise referred to as sequence identity. Sequence identity is frequently measured in terms of percentage identity (or similarity or homology); the higher the percentage, the more similar the two sequences are.

Methods of alignment of sequences for comparison are well known in the art. Various programs and alignment algorithms are described in: Smith and Waterman (Adv. Appl. Math. 2: 482, 1981); Needleman and Wunsch (J. Mol. Biol. 48:443,1970); Pearson and Lipman (PNAS. USA 85: 2444, 1988); Higgins and Sharp (Gene, 73: 237-244, 1988); Higgins and Sharp (CABIOS 5:151-153, 1989); Corpet et al. (Nuc. Acids Res. 16:10881-10890,1988); Huang et al. (Comp. Appls Biosci. 8: 155-165,1992); and Pearson et al. (Meth. Mol. Biol. 24: 307-31, 1994). Altschul et al. (Nature Genet., 6: 119-129, 1994) presents a detailed consideration of sequence alignment methods and homology calculations.

The alignment tools ALIGN (Myers and Miller, CABIOS 4:11-17, 1989) or LFASTA (Pearson and Lipman, 1988) may be used to perform sequence comparisons (Internet Program **©** 1996, W. R Pearson and the University of Virginia, "fasta20u63" version 2.0u63, release date December 1996). ALIGN compares entire sequences against one another, while LFASTA compares regions of local similarity. These alignment tools and their respective tutorials are available on the Internet at the NCSA Website. Alternatively, for comparisons of amino acid sequences of greater than about 30 amino acids, the "Blast 2 sequences" function can be employed using the default BLOSUM62 matrix set to default parameters, (gap existence cost of 11, and a per residue gap cost of 1). When aligning short peptides (fewer than around 30 amino acids), the alignment should be performed using the "Blast 2 sequences" function, employing the PAM30 matrix set to default parameters (open gap 9, extension gap 1 penalties). The BLAST sequence comparison system is available, for instance, from the NCBI web site; see also Altschul et al., J. Mol. Biol. 215:403-410, 1990; Gish. & States, Nature Genet. 3:266-272, 1993; Madden et al. Meth. Enzymol. 266:131-141,1996; Altschul et al., Nucleic Acids Res. 25:3389-3402, 1997; and Zhang & Madden, Genome Res. 7:649-656, 1997.

Orthologs (equivalent to proteins of other species) of proteins are in some instances characterized by possession of greater than 75% sequence identity counted over the full-length alignment with the amino acid sequence of specific protein using ALIGN set to default parameters. Proteins with even greater similarity to a reference sequence will show increasing percentage identities when assessed by this method, such as at least 80%, at least 85%, at least 90%, at least 92%, at least 95%, or at least 98% sequence identity. In addition, sequence identity can be compared over the full length of one or both binding domains of the disclosed fusion proteins.

When significantly less than the entire sequence is being compared for sequence identity, homologous sequences will typically possess at least 80% sequence identity over short windows of 10-20, and may possess sequence identities of at least 85%, at least 90%, at least 95%, or at least 99% depending on their similarity to the reference sequence. Sequence identity over such short windows can be determined using LFASTA; methods are described at the NCSA Website. One of skill in the art will appreciate that these sequence identity ranges are provided for guidance only; it is entirely possible that strongly significant homologs could be obtained that fall outside of the ranges provided. Similar homology concepts apply for nucleic acids as are described for protein.

An alternative indication that two nucleic acid molecules are closely related is that the two molecules hybridize to each other under stringent conditions. Stringent conditions are sequence-dependent and are different under different environmental parameters. Generally, stringent conditions are selected to be about 5°C to 20°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Conditions for nucleic acid hybridization and calculation of stringencies can be found in Sambrook et al. (In Molecular Cloning: A Laboratory Manual, Cold Spring Harbor, New York, 1989) and Tijssen (Laboratory Techniques in Biochemistry and Molecular Biology Part I, Ch. 2, Elsevier, New York, 1993).

Nucleic acid sequences that do not show a high degree of identity may nevertheless encode similar amino acid sequences, due to the degeneracy of the genetic code. It is understood that changes in nucleic acid sequence can be made using this degeneracy to produce multiple nucleic acid sequences that each encode substantially the same protein.

**Specific binding agent:** An agent that binds substantially only to a defined target. Thus a peptide-specific binding agent binds substantially only the defined peptide, or a peptide region within a protein, such as a fusion protein. As used herein, the term "[X] specific binding agent," where [X] refers to a specific protein or peptide, includes anti-[X] antibodies (and functional fragments thereof) and other agents (such as soluble receptors) that bind substantially only to [X]. It is contemplated that [X] can be a family of closely-related proteins (for instance, closely-related TGF-βs) that are recognized by one specific binding agent. An antibody is one example of a specific binding agent

**Subject:** Living multi-cellular vertebrate organisms, a category that includes both human and non-human mammals.

**TGF-β family of proteins:** A family of secreted signaling molecules involved in a number of cellular and developmental processes in eukaryotic cells, including inflammation, immune surveillance, and neoplasia. Members of the TGF-β family of proteins include, but are not limited to: TGF-β2, TGF-β3, TGF-β1, TGF-β4, (chicken), TGF-β5 (*Xenopus*)*,* GDF-9 (mouse/human), BMP-16/nodal (mouse), Fugacin (*Xenopus*)*,* BMP3, Sumitomo-BIP/GDF-10 (mouse), ADMP *(Xenopus),* BMP-9, Dorsalin-1 (Chicken), BMP-10, BMP-13/GDF-6 (mouse), Radar. (Zebrafish), GDF-1/CDMP-1 (mouse/human), BMP-12/GDF-7 (mouse), BMP-5, BMP-6, BMP-7/OP-1, BMP-8/OP-2, PC8/OP-3 (mouse), 60A (*Drosophila*)*,* BMP-2, BMP-4, Decapentaplegic (*Drosophila*)*,* Vg-*1* (*Xenopus*)*,* Univin (sea urchin), Vgr-2/GDF-3, GDF-1, Screw (*Drosophila*)*,* BMP-11, GDF-8, ActivinβC, ActivinβD *(Xenopus*)*,* ActivinβE, BMP-14/GDF-12, ActivinpβA, ActivinβB, GDF-14, Mullerian inhibiting substance, and α-inhibin. The term "TGF-β" is used generally herein to mean any isoform of TGF-β, provided the isoform has immunosuppressive activity. Methods are disclosed herein of using agents to block the immunosuppressive effects of TGF-β.

The term **TGF-βfamily protein function** includes all functions that are associated with a TGF-β family protein, including for instance secondary folding of each TGF-β monomer, tertiary association between the members of the multimeric (for example, homodimeric) TGF-β complex, maturation by cleavage and/or removal of the pro-region (LAP), secretion of the protein from the cell in which it was translated, specific receptor binding, and down-stream activities that result from the binding of a TGF-β family ligand protein with its cognate receptor(s). Such downstream activities include (depending on the TGF-β family member examined and the system used), for instance, regulation of cell growth (proliferation), stimulation of cell growth or proliferation, stimulation of cell differentiation, inhibition of cell growth or proliferation, regulation of cytokine production, induction of cellular differentiation, cell cycle inhibition, control of adhesion molecule expression, stimulation of angiogenesis, induction of leukocyte chemotaxis, induction of apoptosis, suppression of lymphocyte activation, suppression of inflammation, enhancement of wound healing by mechanisms including, stimulation of synthesis of matrix proteins, regulation of immunoglobulin production, including isotype switch recombination, and suppression of tumorigenesis.

Different members of the TGF-β family have different biological specificities and activities. Specificities of the listed TGF-β family proteins are known to one of ordinary skill in the art. See, for instance, Doetschman, Lab.Anim.Sci. 49:137-143, 1999; Letterio and Roberts, Annu. Rev. Immunol. 16:137-61:137-161, 1998; Wahl.J. Exp. Med. 180:1587-1590, 1994; Letterio and Roberts, J. Leukoc. Biol. 59:769-774, 1996; Piek et al., FASEB J. 13:2105-2124, 1999; Heldin et al., Nature 390:465-471, 1979; and De Caestecker et al., J. Nat'l Cancer Inst., 92:1388-1402, 2000.

TGF-β mutants, including fragments of TGF-β and TGF-β peptides, that retain the ability to bind a TGF-β receptor but cannot induce a TGF-β signaling pathway are encompassed by the disclosure. Also encompassed by the disclosure are TGF-β point mutants that retain the ability to bind a TGF-β receptor but cannot induce the TGF-β signaling pathway. Certain TGF-β mutants, such as those disclosed herein, are "neutralizing" molecules.

**TGF-β signaling pathway:** TGF-β transmits a signal across a cell membrane by stimulating the formation of specific heteromeric complexes of type I and type II serine/threonine kinase receptors (for example, a TGF-β receptor). The type II receptors bind ligand (for example, a TGF-β), and phosphorylate and activate the type I receptors, whereas the type I receptors are responsible for the specificity of downstream signaling. The downstream intracellular molecules, or effectors, of the phosphorylated type I receptor are known as Smads.

Smads, the only substrates for type I receptor kinases known to have a signaling function, have two conserved domains, the N-terminal Mad homology 1 and the C-terminal Mad homology 2 domains. Smads are ubiquitously expressed throughout development and in all adult tissues. Functionally, Smads fall into three subfamilies: receptor-activated Smads (R-Smads; Smad1, Smad2, Smad3, Smad5, Smad8), which become activated by type I receptors; common mediator Smads (Co-Smads; Smad4), which oligomerize with activated R-Smads; and inhibitory Smads (I-Smads; Smad 6 and Smad7), which are induced by TGF-β family members.

Activated TGF-β receptors phosphorylate Smad2 and Smad3. Phosphorylation of the C-terminal serine residues in R-Smads by type I receptor kinases is a crucial step in TGF-β signaling. The two most C-terminal serine residues become phosphorylated and, together with a third non-phosphorylated serine residue, form an evolutionarily conserved SSXS motif in all R-Smads. Unphosphorylated Smad proteins exist primarily as monomers, and upon phosphorylation, R-Smads form homo-oligomers, which quickly convert to hetero-oligomers containing the Co-Smad, Smad4.

All R-Smads, mammalian Smad4, and *Xenopus* Smad4α reside in the cytoplasm. However, heteromeric R-Smad/Co-Smad complexes are found in the nucleus, thus the Smads must translocate to the nucleus. The NH1 domains of all eight Smads each contain a lysine-rich motif that, in the case of Smad1 and Smad3, has been shown to function as a nuclear localization signal.

All Smads have transcriptional activity. Heteromeric R-Smad/Co-Smad complexes are the transcriptionally relevant entities *in vivo*. Smad3 and Smad4 bind directly, but with low affinity to Smad binding elements (SBEs), through a conserved β-hairpin loop in the MH1 domain. Additional MH1 sequences, such as α-helix 2, contribute to SBE DNA-binding by Smad3. Because of the low affinity to SBEs, DNA-binding co-factors must be involved in providing a tight and highly specific recognition of the regulatory elements in target genes. The choice of target gene by an activated Smad complex is made by the association of this complex with specific DNA-binding co-factors. Examples of such co-factors include FAST, OAZ, AP-1, TFE3, and AML proteins. Once a Smad complex binds DNA it may control the transcription of target genes, for example by altering nucleosome structure (Massague and Chen, Genes and Development 14:627-644, 2000; Moustakas et al., J. Cell Sci. 114:4359-4369, 2001).

Agents, as disclosed herein, that bind TGF-β, the TGF-β receptor, or any of the TGF-β receptor's downstream signaling partners can block the TGF-β signaling pathway (a blockade of TGF-β signaling). In one embodiment, the agent is a neutralizing agent that results in an inhibition of the activity of the molecule to which it binds. TGF-β mutants, including fragments of TGF-β and TGF-β peptides, which retain the ability to bind a TGF-β receptor but cannot induce the TGF-β signaling pathway are encompassed by the disclosure. Also encompassed by the disclosure are TGF-βpoint mutants that retain the ability to bind a TGF-β receptor but cannot induce the TGF-β signaling pathway. A blockade of TGF-β signaling can prevent, for example, the phosphorylation of a type I receptor, the phosphorylation of a Smad, the binding of a Smad to a Smad binding element, or the transcription of a target gene.

**Therapeutically effective amount of an agent:** A quantity of an agent sufficient to achieve a desired effect in a subject being treated. For instance, when referring to a neutralizing agent such as an anti-TGF-β antibody, this can be the amount necessary to induce a dose-dependent effect. Examples of dose-dependent effects of a neutralizing anti-TGF-β antibody include:
the amount of neutralizing anti-TGF-β antibody that, when administered to a subject, can inhibit an immunosuppressive effect of TGF-β; and
the amount of neutralizing anti-TGF-β antibody that, when administered to a subject following treatment of a tumor, can inhibit recurrence of the tumor.

Other neutralizing agents (for example, chemical compounds, small molecules, or peptides) are clinically relevant and can be administered in a therapeutically effective dose in order to achieve a desired effect in a subject being treated.

An effective amount of an agent may be administered in a single dose, or in several doses, for example daily, during a course of treatment. However, the effective amount of agent will be dependent on the agent applied, the subject being treated, the severity and type of the affliction, and the manner of administration of the agent. For example, a therapeutically effective amount of the neutralizing anti-TGF-β antibody 1D11.16 can vary from about 0.01 mg/kg body weight to about 1. g/kg body weight. In one specific, non-limiting example, a therapeutically effective amount is about 3-4 mg/kg body weight.

The agent disclosed in the present invention have equal application in medical and veterinary settings. Therefore, the general term "subject being treated" is understood to include all animals (for example, humans, apes, dogs, cats, horses, and cows).

**Treatment:** Refers to both prophylactic inhibition of disease (such as tumor recurrence) and therapeutic interventions to alter the natural course of an untreated disease process, such as a tumor growth. Treatment of a tumor include, for instance, the surgical removal of the tumor. Treatment of a tumor can also include chemotherapy, immunotherapy, or radiation therapy. Two or more methods of treating a tumor can be provided to a subject in combination. Treatment of a subject includes inhibiting or measurably reducing the recurrence of a tumor.

**Tumor:** A neoplasm that may be either malignant or non-malignant (benign). Tumors of the same tissue type are tumors originating in a particular organ (such as breast, prostate, bladder or lung). Tumors of the same tissue type may be divided into tumor of different sub-types (a classic example being bronchogenic carcinomas (lung tumors) which can be an adenocarcinoma, small cell, squamous cell, or large cell tumor). Breast cancers can be divided histologically into scirrhous, infiltrative, papillary, ductal, medullary and lobular.

**Tumor recurrence:** The return of a tumor, at the same site as the original (primary) tumor, after the tumor has been removed surgically, by drug or other treatment, or has otherwise disappeared. Tumor recurrence often occurs even though a tumor appears to be completely eradicated (by any method) or has disappeared. However, the eradication is often not complete and, as there exists an established blood supply, a tumor can recur. A subject that has had a tumor removed by any method (for example, surgical removal, drug or other treatment) or that has had a tumor disappear, is at risk for recurrence of a tumor.

Methods are disclosed herein to inhibit a recurrence of a tumor, for instance by any measure amount. The term "inhibits" does not require absolute inhibition. Similarly, the term prevents" does not require absolute prevention. Reducing a recurrence of a tumor includes reducing the recurrence of a tumor by measurable amounts, for example at least 5%, at least 10%, at least 20%, at least 30%, at least 40%, at least 50%, at least 60%, at least 70%, at least 80%, at least 85%, at least 90%,at least 95%, at least 99%, or 100%.

**Vector:** A nucleic acid molecule as introduced into a host cell, thereby producing a transformed host cell. A vector may include nucleic acid sequences that permit it to replicate in a host cell, such as an origin of replication. A vector may also include one or more selectable marker genes and other genetic elements known to those of ordinary skill in the art.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of the present disclosure, suitable methods and materials are described below. In case of conflict, the present specification, including explanations of terms, will control. In addition, the materials, methods, and examples are illustrative only and not intended to be limiting.

### III. Description of Several Specific Embodiments

This disclosure provides antibodies as defined in the claims for use in a method of inhibiting or reducing a recurrence of a tumor in a subject (for instance, a human subject), which method involves administering a therapeutically effective amount of the antibody to the subject in order to block the immunosuppressive effect of TGF-β, wherein the subject is at risk for recurrence of the tumor and wherein the antibody neutralizes an activity of transforming growth factor (TGF)-β, thereby inhibiting or reducing the recurrence of the tumor.

The agent is a monoclonal antibody and the monoclonal antibody is specific for a TGF-β such as the monoclonal antibody obtained from hybridoma 1 D 11.16 (ATCC Accession No. HB 9849). For instance, the anti-TGF-β antibody in some examples inhibits TGF-β from binding a TGF-β receptor.

The tumor referred to in the methods provided herein may be benign or malignant. For instance, the tumor can include a carcinoma, a sarcoma, a leukemia, a lymphoma, or a tumor of the nervous system. In other examples, the tumor includes a breast tumor, a liver tumor, a pancreatic tumor, a gastrointestinal tumor, a colon tumor a uterine tumor, a ovarian tumor, a cervical tumor, a testicular tumor, a brain tumor, a skin tumor, a melanoma, a retinal tumor, a lung tumor, a kidney tumor, a bone tumor, a prostate tumor, a nasopharygeal tumor, a thryoid tumor, a leukemia, or a lymphoma.

Agents used in methods described can be administered, for instance, intravenously, subcutaneously, intradermally, or intramuscularly.

Also described herein are methods of inhibiting or reducing a recurrence of a tumor in a subject, wherein administering the therapeutically effective amount of the agent results in a lack of, or measurable reduction in, tumor growth *in vivo* or *in vitro.*

In further example methods of inhibiting or reducing a recurrence of a tumor in a subject, blocking the immunosuppressive effect of the TGF-β results in increased immunosurveillance by lymphocytes (for instance, T cells or B cells, or a mixture of both) of the subject. For instance, the lymphocytes in some methods include T cells, and the T cells are cytotoxic T lymphocytes (CTL), CD8⁺ cells, CD4⁺ cells, CD4⁺ CD1d-restricted T cells, natural killer cells, or a combination of two or more thereof.

Increased immunosurveillance can be measured as an increased biological activity of the lymphocyte, for instance measured by a CTL assay (for example, a chromium release assay).

Also described are methods of inhibiting or reducing a recurrence of a tumor in a subject, which methods involve contacting a TGF-β receptor with the agent, thereby neutralizing the activity of the TGF-β.For example, the agent in such methods can be an antagonist, an antibody, a small molecule, a chemical compound, a peptide mimetic, a peptide or a protein, or combinations thereof In specific examples of these methods, the agent inhibits TGF-β receptor signaling.

Also described is a method of inhibiting or reducing a recurrence of a tumor in a subject, wherein administering the agent comprises contacting a downstream signaling molecule of the TGF-β receptor with the agent. For example, the agent in such methods can be an antagonist,an antibody, a small molecule, a chemical compound, a peptide mimetic, a peptide or a protein, or combinations thereof. In particular examples of these methods, the downstream signaling molecule includes a Smad protein or a Smad complex DNA-binding co-factor.

Also described is a method of enhancing an activity of an immune cell to inhibit recurrence of a tumor. For instance, such method involves contacting a TGF-β receptor-expressing immune cell with an agent (for example, an antagonist, an antibody, a small molecule, a chemical compound, a peptide mimetic, a peptide or a protein) that blocks a TGF-β signaling pathway, wherein blocking the TGF-β signaling pathway results in increased tumor immunosurveillance by the TGF-β receptor-expressing immune cell, thereby enhancing the activity of the immune cell to inhibit tumor recurrence. By way of example, the TGF-β receptor-expressing immune cell in this method can be a T cell or a B cell In specific examples, the TGF-β receptor-expressing immune cell is a lymphocyte. For instance, it is a T cell, which T cell is a CTL, a CD8⁺ cell, a CD4⁺ cell, a CD4⁺ CD1d-restricted T cell, or a natural killer cell.

In specific contemplated examples of these methods, contacting a TGF-β receptor-expressing immune cell with an agent involves contacting a TGF-β or a TGF-β receptor, or contacting both. By way of example, contracting TGF-β can include contacting with an anti- TGF-β monoclonal antibody, for instance one that is obtained from hybridoma 1D11.16 having ATCC Accession No. HB 9849.

Also described is a method of enhancing an immune response (for instance,a T cell response) in a subject (such as a human) to inhibit recurrence of a tumor, which method involves administering to the subject a therapeutically effective amount of an agent that blocks a TGF-β signaling pathway, wherein blocking the TGF-β signaling pathway results in increased tumor immunosurveillance in the subject, thereby enhancing the immune response in the subject to inhibit recurrence of a tumor. By way of example, the immune response can be a T cell response, which T cell response includes a CTL response, a CD8⁺CTL response, a CD4⁺ T cell response, a CD4⁺ CD1d-restricted T cell response or a natural killer cell response. In specific methods, the cytotoxic T cell response is CTL-mediated immumosurveillance. Agents for use in each of these methods can be an antagonist, an antibody, a small molecule, a chemical compound, a peptide mimetic, a peptide or a protein.

In specific examples of these methods of enhancing an immune response in a subject, the agent contacts a TGF-β or a TGF-β receptor. For instance, in some instances the agent includes an anti-TGF-β monoclonal antibody that is obtained from hybridoma 1D 11.16 (ATCC Accession No. HB 9849).

Described herein is a method for screening for an agent that inhibits or reduces tumor recurrence, which method involves contacting a TGF-β receptor-expressing immune cell with TGF-β;contacting the TGF-β receptor-expressing immune cell with an agent; and assaying for a decrease in activity of TGF-β signaling in the TGF-β receptor-expressing immune cell, as compared to a TGF-β receptor-expressing control immune cell, wherein the control immune cell is not contacted with the agent, thereby screening for an agent that inhibits or reduces tumor recurrence.

In further examples of such methods for screening, the method further involves assaying for an increase in activity of the TGF-β receptor-expressing immune cell, for instance a CTL. Assaying the activity of a CTL can included, for instance, measurement using a CTL assay such as those described herein.

In still further examples of screening for an agent that inhibits or reduces tumor recurrence, the decrease in activity of TGF-β signaling includes decreased phosphorylation of a Smad protein, decreased nuclear translocation of a Smad protein, or decreased DNA binding of a Smad complex.

Alternatively, the increase in activity of the TGF-β receptor-expressing immune cell comprises increased immunosurveillance. By way of example, such increased immunosurveillance can include in some instances increased CTL activity.

### IV. Method of Inhibiting Tumor Recurrence by Blocking the TGF-β Signaling Pathway

Methods are disclosed herein of inhibiting a recurrence of a tumor in a subject by blocking the TGF-β signaling pathway in order to block TGF-β's immunosuppressive effects. Methods of enhancing the activity of an immune cell by blocking the TGF-β signaling pathway are also disclosed. Immune cells that are susceptible to a block in the TGF-β signaling pathway are those cells that express the TGF-β receptor. The disclosure also provides methods of enhancing an immune response in a subject by blocking the TGF-β signaling pathway. Any of the agents disclosed herein can be used to block the TGF-β signaling pathway.

### Inhibiting Tumor Recurrence by Blocking the TGF-β Signaling Pathway

The mechanism of down-regulation of tumor immunosurveillance by CTLs, caused by the immunosuppressive effects of TGF-β on CTLs, has been studied using a mouse tumor model in which tumors shows a growth-regression-recurrence pattern after tumor inoculation (Matsui et al., J. Immunol. 163:184, 1999). With this mouse tumor model, it was demonstrated that tumor recurrence was the result of incomplete elimination of tumor cells by CTLs that were negatively regulated by IL-13 produced by CD4⁺ CD1d-restricted NKT cells through the IL-4Rα-STAT6 signaling pathway (Terabe et al., Nature Immunol. 1:515, 2000).

It is disclosed herein that IL-13 made by CD4⁺CD1d-restricted NKT cells induces CD1 1b⁺Gr-1⁺ non-lymphoid cells of myeloid origin to produce TGF-β. It is also disclosed herein that TGF-β causes the down-regulation of CD8⁺ CTL-mediated tumor immunosurveillance (FIG. 10). Thus, methods are disclosed herein of inhibiting a recurrence of a tumor in a subject by blocking TGF-β's immunosuppressive effects. The method includes administering to a subject a therapeutically effective amount of an agent which, for example, blocks TGF-β binding to the TGF-β receptor, thereby blocking the TGF-β signaling pathway. Administration of an agent which blocks the TGF-β signaling pathway is particularly effective against tumors that have escaped CTL immunosurveillance as a result of the immunosuppressive effects of TGF-β.

### Enhancing an Activity of an Immune Cell by Blocking the TGF-β Signaling Pathway

The disclosure provides methods of enhancing the activity of an immune cell by blocking the TGF-β signaling pathway. Immune cells that are susceptible to a block in the TGF-β signaling pathway are those cells that express the TGF-β receptor. Any of the agents disclosed herein can be used to block the TGF-β signaling pathway.

A method is disclosed herein for enhancing an activity of an immune cell. Immune cells include leukocytes (for instance, neutrophils, eosinophils, monocytes, basophils, macrophages, B cells, T cells, dendritic cells, and mast cells), as well as other types of cells involved in an immune response. The method includes contacting an immune cell that expresses a TGF-β receptor with an agent which blocks the TGF-β signaling pathway. In one embodiment, the immune cell is a lymphocyte, such as a T cell or a B cell. In other embodiments, the immune cell is a CTL, a CD8⁺ CTL, a CD4⁺ T cell, a CD4⁺ CD1d-restricted T cell, an NK cell, or an NKT cell. In, a further embodiment, the immune cell is a granulocyte. The immune cell can be either *in vivo* or *in vitro.* The agent can either bind TGF-β, a TGF-β receptor or a TGF-β receptor downstream signaling molecule.

In one embodimen the activitiy of an immune cell is enhanced in a subject, following the administration of an agent as defined in the claims which bocks the TGF-β signaling pathway. Immune cells having an enhanced activity, for example increased tumor immunosurveillance, following the administration of the agent include cells that express a TGF-β receptor, such as a CTL. In one embodiment, the immune cell with the enhanced activity is in a subject suffering from a tumor that has escaped CTL immunosurveillance. In another embodiment, an enhanced activity of an immune cell, such as enhanced CTL immunosurveillance, inhibits the recurrence of a tumor.

### Enhancing an Immune Response in a Subject by Blocking the TGF-β Signaling Pathway

The disclosure provides methods of enhancing an immune response in a subject by blocking the TGF-β signaling pathway. In one embodiment, an enhanced immune response, for example increased tumor immunosurveillance, inhibits the recurrence of a tumor. Any of the agents disclosed herein can be used to block the TGF-β signaling pathway, thereby enhance an immune response.

A method is disclosed herein for enhancing an immune response in a subject. The method includes administering to the subject a therapeutically effective amount of an agent, which blocks the TGF-β signaling pathway, to enhance the immune response. In one embodiment, the immune response is a T cell response. In another embodiment, the immune response involves a TGF-β receptor-expressing cell. The cell expressing a TGP-β receptor can be, but is not limited to, a CTL a CD8⁺ CTL, a CD4⁺ T cell, a CD4⁺ CD1d-restricted T cell, an NK cell, or an NKT cell. In a further embodiment, the immune response is CTL-mediated immunosurveillance. In one embodiment, a subject with an enhanced immune response is suffering from a tumor that has escaped CTL immunosurveillance. In another embodiment, an enhanced immune response inhibits the recurrence of a tumor in a subject

A method is also disclosed herein for enhancing a T cell-mediated immune response. The method includes administering to the subject a therapeutically effective amount of an agent, which blocks the TGF-β signaling pathway, to improve a T cell-mediated immune response. In one embodiment, the T cell-mediated immune response is CTL-mediated immunosurveillance. In another embodiment, the T cell-mediated immune response is an NKT cell response. In a further embodiment, T cell-mediated immune response is a CD4⁺ CD1d-restricted T cell response.

### Agents that Block the TGF-β Signaling Pathway

Agents, including neutralizing agents, that block the TGF-β signaling pathway are disclosed herein. Also disclosed herein are agents that block the immunosuppressive effects of TGF-β, thus these agents enhance an activity of an immune cell, such as CTL immunosurveillance, or an immune response in a subject. In one embodiment, an agent inhibits the recurrence of a tumor that has escaped CTL immunosurveillance.

The agent can be any substance, including, but not limited to, an antagonist, an antibody, a chemical compound, a small molecule, a peptide mimetic, a peptide or a protein. The agent is preferably a non-toxic agent. An agent can be, for example, an enzyme (for example, a kinase or a phosphorylase) or another catalytic molecule that selectively binds and alters the function of a protein and/or the activity of a molecular pathway, such as the TGF-β signaling pathway. For example, proteins can be functional when phosphorylated and nonfunctional when de-phosphorylated. A functional, phosphorylated, protein can become nonfunctional when exposed to a de-phosphorylating agent such as a phosphorylase. Thus, a cell that is active as the result of expressing a functional protein, can become inactivated when it is in contact with an agent that inhibits (neutralizes) the function of the protein. The reverse is also true. For example, a cell that is inactive as the result of expressing a functional protein, can become activated when it is in contact with an agent that inhibits (neutralizes) the function of the protein.

In the embodiments the agent is a neutralizing agent as defined in the claims. An agent can neutralize (inhibit an activity of) a molecule by specifically binding it, thereby preventing the molecule from performing at least one function. For example, a neutralizing agent can prevent a molecule from interacting with other molecules. In one specific, non-limiting example, a neutralizing agent prevents TGF-β from specifically binding the TGF-β receptor. TGF-β activity can be neutralized with any of the agents disclosed herein.

In one embodiment, the agent is an antagonist. An antagonist is any substance that tends to nullify, or neutralize, the action of another, for example a drug that binds to a receptor, such as a TGP-β receptor, without eliciting a biological response. In one embodiment, the antagonist is a chemical compound that neutralizes TGF-β directly. Also described herein is a chemical compound that neutralizes the TGF-β receptor- or at least one of its downstream signaling molecules (for example, Smad 2, Smad3, or Smad 4), or a Smad complex DNA-binding co-factor.

In one embodiment, the agent interacts (for example, specifically binds) with the TGF-β molecule directly. The agent is an anti-TGF-β antibody. The anti-TGF-β antibody is a monoclonal antibody obtained from the hybridoma 1D11.16 (ATCC Accession No. HB 9849) binds TGF-β. Agents, such as the 1D11.16 antibody, can bind TGF-β and neutralize its activity by preventing it from binding the anti-TGF-β receptor.

Alternatively, an agent can form a complex with a ligand, such as TGF-β so that it is still capable of binding a receptor, such as a TOF-β receptor, but the ligand:agent complex is incapable of activating the receptor and transmitting a signal.

An agent can specifically bind a receptor, such as the TGF-β receptor, and prevent the receptor from transmitting a signal across the cell membrane into the cell. More specifically, an agent can specifically blind a receptor, such as the TGF-β receptor, at its ligand-binding site thereby preventing a ligand, such as TGF-β from binding to the receptor. As disclosed herein, TGF-β is used generally herein to mean any isoform of TGF-β, provided the isoform has immunosuppressive activity. In one specific, non-limiting example, the agent is an anti- TGF-β receptor antibody. In another specific, non-limiting example, the agent is a TGF-β mutant. TGF-β mutants include fragments of TGF-β and TGF-β peptides that retain the ability to bind a TGF-β receptor but cannot induce the TGF-β signaling pathway. TGF-β mutants also include TGF-β point mutants that retain the ability to bind a TGF-β receptor but cannot induce the TGF-β signaling pathway, or induce it only at a low level compared to the wildtype TGF-β.

An agent can also specifically bind one or more of the receptor's downstream signaling molecules. For example, some agents neutralizes TGF-β activity by specifically binding a downstream signaling molecule and preventing the transmission of an intracellular TGF-β signal. TGF-β downstream signaling molecules include, but are not limited to, Smad2, Smad3, Smad4, or Smad complex DNA-binding co-factors.

A neutralizing agent may be soluble TGF-β receptor. The soluble TGF-β receptor specifically binds TGF-β and competes with the TGF-β cell surface receptor for any available TGF-β. Preventing TGF-β from binding its endogenous receptor neutralizes the activity of TGF-β. provided that sufficient soluble TGF-β receptor is present in order to bind all of the available TGF-β ligand.

The TGF-β receptor can be expressed in a lymphocyte, such as a T lymphocyte. More specifically, the TGF-β receptor can be expressed in a CTL. Thus, the method of using an agent to neutralize the activity of TGF-β prevents TGF-β signaling in a TGF-β receptor -expressing CTL.

### Measurable Effects of Agents that Block the TGF-β Signaling Pathway

In one embodiment, the method of administering an agent that blocks the TGF-β signaling pathway results in a change in CTL activity. The change in CTL activity can be measured in any number of ways known to one of skill in the art. CTL activity can be monitored by measuring production of some chemokines, for example RANGES, or the production of IFN-γ by ELISA, ELISPOT (enzyme-linked immunospot) or RT-PCR. Other methods include intracellular cytokine staining in combination with detection of the MHC class I-tetramer and a CTL assay. One specific, non-limiting example of a CTL assay is the chromium release assay. In the chromium release assay, target cells expressing an antigen, for example the P18-IIIB peptide, on their surface are labeled with a radioactive isotope of chromium (⁵¹Cr). Cell of interest, such as CD8⁺ T cells, are then mixed with the target cell, either in the presence or absence of a cytokine, and incubated for several hours. Specific, non-limiting examples of cytokines include IL-4, IL-13, and TGF-β. Lysis of antigen-expressing cells releases ⁵¹Cr into the medium. Antigen-specific lysis is calculated by comparing lysis of target cells expressing the antigen or control antigens in the presence or absence of effector cells, and is usually expressed as the percent antigen-specific lysis.

The change in activity, as a result of administration of an agent, can be an increase or a decrease in CTL activity. The change in CTL activity can be measured as at least about a two-fold, at least about a five-fold, at least about a ten-fold, at least about a twenty-fold, at least about a fifty-fold, at least about a one hundred-fold, or at least about a two-hundred fold increase or decrease in CTL activity.

### Administration of Agents that Block the TGF-β Signaling Pathway

The agents disclosed herein can be administered to a subject thought to be a candidate for tumor recurrence. In some embodiments, the agent is administered to a subject prior to treatment of a tumor. Treatment of the tumor includes, but is not limited to, surgical removal of the tumor, chemotherapy, immunotherapy or radiation therapy. In other embodiments, the agent is administered to the subject simultaneously with treatment of the tumor, or following treatment of the tumor. In further embodiments, the agent is administered to the subject in combination with at least one additional agent either prior to, simultaneously with, or following treatment of the tumor. The additional agent can also be another agent described herein that inhibits or reduces tumor recurrence. Alternatively, the additional agent can be an agent that improves the subject's ability to inhibit a tumor recurrence or an agent (such as an antibiotic) that helps the subject fight infection during the course of treatment for the tumor. For example, the additional agent can be an agent that stimulates the immune system, such as a cytokine.

In general, the agent is administered to a subject in an amount sufficient to inhibit the recurrence of a tumor at the original site of the primary tumor growth. In another embodiment, the agent is administered to a subject in an amount sufficient to inhibit an immunsuppressive effect of TGF-β. The amount administered to a subject can vary from about 0.01 mg/kg body weight to about 1 g/kg body weight. In one specific, non-limiting example, the amount administered is about 3-4 mg/kg body weight.

The method of inhibiting tumor recurrence can be applied to a subject suffering from any type of tumor. In one embodiment, the tumor is susceptible to an immune response. In another embodiment, the tumor is susceptible to a T cell-mediated immune response. In a further embodiment, the tumor is susceptible to a CTL-mediated immune response. In yet another embodiment, the tumor is susceptible to immunosurveillance, such as a melanoma, a renal tumor, a breast tumor, a colon tumor or an osteosarcoma.

The tumor can be a benign tumor or a malignant tumor. The tumor can include a carcinoma, a sarcoma, a leukemia, a lymphoma, or a tumor of the nervous system. In several specific, non-limiting embodiments, the tumor includes a breast tumor, a liver tumor, a pancreatic tumor, a gastrointestinal tumor, a colon tumor, a uterine tumor, a ovarian tumor, a cervical tumor, a testicular tumor, a prostate tumor, a brain tumor, a skin tumor, a melanoma, a retinal tumor, a lung tumor, a kidney tumor, a bone tumor, an osteosarcoma, a nasopharygeal tumor, a thryoid tumor, a leukemia, or a lymphoma.

The subject can be a mammal such as a human, a monkey, a horse, a cow, a pig, a dog, a cat, a mouse or a rat.

### V. Method of Screening for an Agent that Inhibits Tumor Recurrence

Methods are descrided for screening agents that inhibits tumor recurrence. In such methods, an immune cell expressing the TGF-β receptor is contacted with an agent (a compound) of interest, and the effect of the agent on the immune cell or on TGF-β signaling is then assayed. A decrease in TGF-β signaling, for example a decrease in phosphorylation of downstream signaling molecule, or a decrease in Smad complex DNA binding indicates that the compound is an agent that blocks the TGF-β signaling pathway. Similarly, an agent is identified as one that blocks the TGF-β signaling pathway if an immune cell, whose activity is normally inhibited by TGF-β, demonstrates an increase in activity or a prolonged period of activation following contact with the agent.

A method is described for screening for an agent that blocks the TGF-β signaling pathway, using a cell of the immune system. The method includes, in other examples, contacting a TGF-β receptor-expressing immune cell, such as a CTL, with an agent that binds the TGF-β receptor, and assaying the activity of the TGF-β receptor-expressing immune cell as compared to the activity of a TGF-β receptor-expressing control immune cell that has not been contracted with the agent. Examples of activity of a TGF-β receptor-expressing immune cell include cell lysis, cell proliferation, cytokine production, inhibition of tumor growth or inhibition of tumor recurrence. The method also includes contacting a TGF-β receptor-expressing immune cell, such as a CTL, with an agent that binds the TGF-β receptor, and assaying the activity of the TGF-β receptor signaling pathway in the cell and comparing it to the TGF-β receptor signaling pathway in a control cell that has not been contacted with the agent. The screening method can be performed in single vessels, or in arrays of vessels, such as in a high-throughput assay.

The method may include contacting a TGF-β receptor-expressing immune cell, such as a CTL, with the combination of TGF-β and an agent that binds TGF-β, and assaying the activity of the TGF-β receptor-expressing immune cell as compared to a TGF-β receptor-expressing control immune cell that has not been contracted with the agent Examples of activity of a TGF-β receptor-expressing immune cell include cell lysis, cell proliferation, cytokine production, inhibition of tumor growth or inhibition of tumor recurrence.

Measuring a blockade of TGF-β signaling, and thus a change in activity of a cell expressing a TGF-β receptor, can be assessed by any means known to one of skill in the art. In one embodiment, western blot analysis is used to measure the level of a protein in a cell incubated in the presence or absence of an agent, or the level of protein phosphorylation in a cell incubated in the presence or absence of an agent. In another embodiment, a luciferase assay is used to measure the effectiveness of the TGF-β signaling pathway to activate gene expression in a cell that is incubated in the presence or absence of an agent. Alternatively, northern blot analysis can be used to measure the up-regulation or down-regulation of gene products in cells incubated in the presence or absence of an agent. TGF-β signaling also can be measured using cell proliferation assays of TGF-β-dependent cell lines.

In one specific, non-limiting example, measuring a blockade of TGF-β signaling is assessed by measuring CTL function. In some instances CTL activity is increased in the presence or absence of a TGF-β blocking agent. CTL activity can be measured using any method known to one of skill in the art, for instance a CTL assay, such as a chromium release assay.

The effects of a blockade of TGF-β signaling also can be measured, for instance by examining the formation or recurrence of tumors, or the relative rate of growth of a recurring tumor in a subject following the administration of an agent to a subject, such as a mouse. The recurrence of a tumor can be examined in the presence or absence of an agent prior to, during, or following the treatment of the primary tumor. Treatment of the primary tumor includes surgical removal of the tumor, chemotherapy, immunotherapy, or radiation therapy. Thus, among other uses, functional assays of agents that block the TGF-β signaling pathway permit optimization of the dosage amounts of each agent effective in therapeutic uses, such as inhibiting a tumor recurrence. These assays also can be used to test known agents, as well as newly identifies agents putative agents, or combinations of these agents for their ability to block the immunosuppressive effects of TGF-β. Candidate agents can initially be screened for subsequent selection and testing in one or more of the assays described herein.

### VI. Pharmaceutical Compositions and Administration

Agents that inhibit tumor recurrence, including the 1D11.16 anti- TGF-β neutralizing monoclonal antibody and other agents effective at blocking the TGF-β signaling pathway, can be administered directly to the subject for the inhibition of tumor recurrence. Pharmaceutical compositions that include an active agent can be formulated with an appropriate solid or liquid carrier, depending on the particular mode of administration chosen. The pharmaceutically acceptable carriers and excipients useful in this disclosure are conventional. For instance, parenteral formulations usually comprise injectable fluids that are pharmaceutically and physiologically acceptable fluid vehicles such as water, physiological saline, other balanced salt solutions, aqueous dextrose, glycerol or the like. Excipients that can be included are, for instance, other proteins, such as human serum albumin or plasma preparations. If desired, the pharmaceutical composition to be administered can also contain minor amounts of non-toxic auxiliary substances, such as wetting or emulsifying agents, preservatives, and pH buffering agents and the like, for example sodium acetate or sorbitan monolaurate.

The dosage form of the pharmaceutical composition will be determined by the mode of administration chosen. For instance, in addition to injectable fluids, topical and oral formulations can be employed. Topical preparations can include eye drops, ointments, sprays and the like. Oral formulations can be liquid (for example, syrups, solutions or suspensions), or solid (for example, powders, pills, tablets, or capsules). For solid compositions, conventional non-toxic solid carriers can include pharmaceutical grades of mannitol, lactose, starch, or magnesium stearate. Actual methods of preparing such dosage forms are known, or will be apparent, to those skilled in the art.

The agents of this disclosure can be administered to humans or other animals on whose cells they are effective in various manners such as topically, orally, intravenously, intramuscularly, intraperitoneally, intranasally, intradermally, intrathecally, and subcutaneously. The particular mode of administration and the dosage regimen will be selected by the attending clinician, taking into account the particulars of the case (for example, the subject, the disease, the disease state involved, and whether the treatment is prophylactic). Treatment can involve daily or multi-daily doses of compound(s) over a period of a few days to months, or even years.

The pharmaceutical compositions that comprise an agent, such as the 1D11.16 anti-TGF-β neutralizing monoclonal antibody and other agents effective at blocking the TGF-β signaling pathway, in some embodiments of the disclosure will be formulated in unit dosage form, suitable for individual administration of precise dosages. For example, a therapeutically effective amount of the 1D11.16 anti-TGF-β neutralizing monoclonal antibody can vary from about 0.1 mg/Kg body weight to about 1 g/Kg body weight. An effective amount of an agent, such as the 1D11.16 anti-TGF-β neutralizing monoclonal antibody, can be administered in a single dose, or in several doses, for example daily, during a course of treatment. The amount of active compound(s) administered will be dependent on the agent being used, the subject being treated, the severity of the affliction, and the manner of administration, and is best left to the judgment of the prescribing clinician. An effective amount of an agent can be administered prior to, simultaneously with, or following treatment of a tumor. Within these bounds, the formulation to be administered will contain a quantity of the active components) in amounts effective to achieve the desired effect in the subject being treated, for instance to measurably reduce the recurrence of a tumor.

A therapeutically effective amount of an agent, such as the 1D11.16 anti-TGF-β neutralizing monoclonal antibody, can be the amount of agent necessary to inhibit the recurrence of a tumor or the amount necessary to measurably reduce the recurrence of a tumor. In some embodiments, a tumor suppressive amount of an agent is an amount sufficient to inhibit or reduce the recurrence of a tumor (for instance, any of the tumor suppressive amounts discussed herein) without causing a substantial cytotoxic effect (for example, without killing more than 1%, 2%, 3%, 5%, or 10% of normal cells in a sample).

Site-specific administration of the disclosed compounds can be used, for instance by applying an agent, such as the 1D11.16 anti-TGF-β neutralizing monoclonal antibody, to a region of tissue from which a tumor has been removed or near a region of tissue from which a tumor has been removed. In some embodiments, sustained intra-tumoral (or near-tumoral) release of the pharmaceutical preparation that comprises a therapeutically effective amount of an agent, such as the 1D11.16 anti- TGF-β neutralizing monoclonal antibody, may be beneficial. Slow-release formulations are known to those of ordinary skill in the art. By way of example, polymers such as bis(p-carboxyphenoxy)propane-sebacic-acid or lecithin suspensions may be used to provide sustained intra-tumoral release.

It is specifically contemplated in some embodiments that delivery is via an injected and/or implanted drug depot, for instance comprising multi-vesicular liposomes such as in DepoFoam (SkyePharma, Inc, San Diego, CA) (see, for instance, Chamberlain et al., Arch. Neuro. 50:261-264, 1993; Katri et al., J. Pharm. Sci. 87:1341-1346, 1998; Ye et al., J. Control Release 64:155-166, 2000; and Howell, Cancer J. 7:219-227, 2001).

The invention is illustrated by the following non-limiting Examples.

### EXAMPLE 1

### Evaluating the Direct Effect of IL-13 on CTL in vitro

In BALB/c mice injected subcutaneously with the fibrosarcoma 15-12RM, tumors show a growth-regression-recurrence pattern. The spontaneous regression was mediated by CD8⁺ CTL (Matsui et al., J. Immunol. 163:184-193, 1999). As was shown previously, both IL-13 and CD4⁺ CD1d-restricted T cells, which are most likely NKT cells as they are the only known CD1d-restricted T cells in the mouse (Kronenberg and Gapin, Nat. Rev. Immunol., 2:557-568, 2002), were necessary to down-regulate this CTL-mediated tumor immunosurveillance (Terabe et al., Nature Immunol. 1:515-520,2000). To understand the mechanism of down-regulation of tumor immunosurveillance induced by IL-13, the direct effect of IL-13 on CTL *in vitro* was initially examined.

### Methods

Spleen cells from BALB/c mice previously immunized with 1 × 10⁷ PFU of recombinant vaccinia vPE16, which expresses HIV gyp160, were stimulated with 1x10⁶ rad-irradiated naive BALB/c spleen cells pulsed with 1 µM P18-IIIB peptide in complete T cell medium (CTM) supplemented IL-2 (20 U/ml; R&D Systems, Minneapolis, MN) either in the presence or absence of various doses of IL-13 (50 ng/ml, 5 ng/ml, 0.5 ng/ml) (R&D Systems, Minneapolis, MN). The P18 peptide is the immunodominant epitope peptide of the V3 loop of HIV gp160 responsible for much of the CTL activity against the 15-12RM tumor (Matsui et al., J. Immunol. 163:184, 1999). Recombinant mouse IL-2 and IL-13 were obtained from R&D systems, Minneapolis, MA.

After 6 days of culture, viable cells were harvested and used as effector cells for the CTL assay. Cytotoxic activity of CD8⁺ T cells against several target cells, such as 18 Neo fibroblasts, was measured by a four hour-⁵¹Cr-release assay. The 18Neo fibroblast is a BALB/c 3T3-derived cell line transfected with a null vector expressing only the *Neo^{r}* gene as a control. These cells were maintained in CTM which consisted of RPMI1640 with 10% FCS, L-glutamine, sodium pyruvate, nonessential amino acids, penicillin, streptomycin and 5 × 10⁻⁵ M 2-ME, containing 200 µg/ml of geneticin (Sigma, St. Louis, MO).

The percentage of specific ⁵¹Cr release in the CTL assay was calculated as follows: 100 × (experimental release - spontaneous release)/(maximum release - spontaneous release). Maximum release was determined from supernatants of cells that were lysed by addition of 5 % Triton X-100. Spontaneous release was determined from target cells incubated without added effector cells.

### Results

The CTL stimulated in the presence of IL-13 did not show any enhancement or reduction of CTL activity against P18-pulsed target cells (FIG. 1A). IL-13 also had no effect on the cytolytic activity when it was added during the CTL assay. These results are consistent with published reports that T cells lack IL-13 receptors (Zurawski and de Vries Immunol. Today, 15:19-26, 1994), and suggest that although IL-13 is necessary for down-regulation of CTL tumor immunosurveillance, it could not directly affect CTL induction or activity. Thus, there must be other downstream steps that directly down-regulate CTL activity.

### EXAMPLE 2

### T Cell Transfer Using RAG2 KO and RAG2IL-4Rα Double KO Recipient Mice

since IL-13 could not directly act on CD8⁺ CTL, in order to determine which intermediate cell population responds to IL-13 and then acts on CTL to down-regulate tumor immunosurveillance, a T cell transfer experiment using RAG2 KO and RAG2IL-4Rα double KO recipient mice was performed.

### Methods

Female BALB/c mice were purchased from Charles River Breeding Laboratories (Frederick, MD). RAG2 knockout (KO) mice on a BALB/c background were obtained from Taconic (Germantown, NY). Mice deficient for both RAG2, IL-4Rα (RAG2IL-4Rα double KO) were bred under pathogen-free conditions. All the mice were maintained in a pathogen-free animal facility and were used at 6-10 weeks of age.

Cells require IL-4Rα to as part of the receptor to respond to IL-13. Thus, T cells were negatively isolated from spleen cells of wild-type (RAG2 KO) or IL-4Rα KO BALB/c mice (obtained from Dr. Nancy Noben-Trauth, George Washigton University). Single-cell suspensions of spleen cells were purified by negative selection for cells expressing MHC class II, CD11b, CD11c and DX5 by using magnetic beads (Mylteni Biotech). The latter cell surface marker is expressed on NK but not NKT cells (Kronenberg and Gapin, Nat. Rev. Immuno/., 2:557-568, 2002). The purified T cells contained 2-3% CD1d tetramer positive cells (NKT cells), compared to 1-1.5% in spleen cells.

Fifty million purified T cells were inoculated into RAG2 KO or RAG2IL-4Rα double KO mice intravenously one week prior to 15-12RM injection. 15-12RM is a fibrosarcoma derived from a BALB/c 3T3 fibroblast line transfected with the HIV envelope gp160 gene, mutant k-ras and c-myc (Matsui et al., J. Immunol. 163:184, 1999). The 15-12RM cells were maintained in complete T cell medium (CTM) which consisted of RPMI1640 with 10% FCS, L-glutamine, sodium pyruvate, nonessential amino acids, penicillin, streptomycin and 5 × 10⁻⁵ M 2-ME, containing 200 µg/ml of geneticin (Sigma, St. Louis, MO).

One million 15-12RM cells were injected subcutaneously on the right flank of the mouse. To deplete CD4⁺ cells *in vivo*, mice were inoculated intraperitonealy. with 0.5 mg of anti-CD4 monoclonal antibody (GK1.5; Frederick Cancer Research and Development Center, NCI, Frederick, MD) or control rat IgG (Sigma, St. Louis, MO) three days in a row from day 0 and twice a week thereafter. In some experiments, mice were treated on day 0, 1 and 3 with 0.2 mg of a fusion protein of murine IL-13Rα2 and human IgG1 (sIL-13Rα2-Fc), made by Genetics Institute, as described previously (Donaldson et al., J. Immunol. 161:2317, 1998). Alternatively, the mice were treated every other day for 10 days with 0.1 mg of either anti-TGF-β monoclonal antibody 1D11.16, specific for TGF-β1, -β2, -β3 (Dasch et al., J. Immunol. 142:1536, 1989), or the isotype matched control monoclonal (13C4), by intraperitoneal injection (Strockbine et al., Infect. Immun. 50:695, 1985). 1D11.16 and 13C4 were made by Genzyme (Cambridge, MA).

### Results

RAG2 KO or RAG2/IL-4Rα double KO mice did not reject tumors at all because they lack T and B cells. However, RAG2 KO mice receiving transferred T cells, regardless of the expression of IL-4Rα by the T cells, behaved like wild-type BALB/c mice, implying that both CD8⁺ effector cells and CD1d-restricted T regulatory cells were successfully reconstituted. Further, the fact that T cells from IL-4Rα KO mice reconstitute as well as wild-type T cells implies that neither the effector nor the regulatory T cells themselves need to be able to respond to IL-13. In contrast, in RAG2IL-4Rα double KO mice that received wild-type T cells, the tumor did not recur after initial growth and regression, even though the transferred T cells expressed IL-4Rα (FIG. 2). Since it was shown that IL-4 was neither necessary nor sufficient for down-regulation of tumor immunosurveillance in this system (Terabe et al., Nature Immunol. 1:515, 2000), the cytokine responsible for signaling through IL-4Rα to regulate tumor immunosurveillance was IL-13. Therefore, the cells responding to IL-13 that mediate the down-regulation of tumor immunosurveillance were derived from the RAG2 KO host, which lacks T and B cells. It was concluded that the responder cells for IL-13 are non-T-non-B cells and that they may produce a soluble factor that directly down-regulates CTL activity.

### EXAMPLE 3

### TGF-β1 Suppresses CTL Function

To understand how non-T-non-B cells respond to IL-13 and suppress CTL, induction in tumor-bearing mice, it was considered that TGF-β1 is known for its ability to suppress lytic activity of CD8⁺ T cell function (Mule et al., Cancer Immunol. Immunother. 26:95, 1988).

### Methods

To examine the ability of TGF-β1 to suppress CTL function, spleen cells of vPE16-immunized mice were stimulated *in vitro* for a week with P18 peptide in the presence of various doses of TGF-β (100 ng/ml, 10 ng/ml, 1 ng/ml) and then tested these in a CTL assay (see Example 1, above). Recombinant human TGF-β1 was purchased from Peprotech (Rocky Hill, NJ).

In some experiments, non-T-non-NK cells were prepared from spleen cells from both naïve and 15-12RM-injected BALB/c mice by depleting CD4⁺, CD8⁺, and DX5⁺ cells by magnetic beads conjugated with anti-CD4, anti-CD8, and anti-DX5 antibodies (Miltenyi Biotec; Auburn, CA). After being pulsed with 1 µM P18-IIIB peptide, the cells were admixed in CTM supplemented with with IL-2 (20 U/ml), either in the presence or absence of anti-TGF-β antibody (50 µg/ml, 1D11), with T cells from vPE16-injected mouse spleen cells that were purified with magnetic beads (Miltenyi Biotec; Auburn, CA) as indicated in FIG. 3. After a week of culture, viable cells were harvested and used as effector cells for the CTL assay, as described above.

### Results

When vPE16-immunized spleen cells were stimulated *in vitro* in the presence of TGF-β1, cytolytic activity was suppressed (FIG. 1B). TGF-β had no effect in the lytic assay itself.

The CTL cultured with non-T-non-NK cells from 15-12RM-injected mice showed lower cytolytic activity than those cultured with the cells from naive mice. However, this suppression was fully overcome by adding anti-TGF-β antibody during the culture (FIG 3). These results suggest that, in contrast to IL-13, TGF-β made *ex vivo* by non-lymphoid cells (FIG. 4A and 4B) has the potential to directly suppress CTL induction.

### EXAMPLE 4

### TGF-β1 Production is Up-Regulated in Tumor-Bearing Mice In Vivo

If TGF-β1 is the cytokine induced by IL-13 in tumor-bearing mice to down-regulate tumor immunosurveillance, TGF-β1 production should be increased in non-T and non-B cells of tumor bearing mice, since expression of IL-4Rα, which is a component of IL-13R, was not necessary in T or B cells to down-regulate immunosurveillance. To determine whether TGF-β1 production is up-regulated in tumor-bearing mice *in vivo*, non-lymphoid cells that were negative for Thy 1.2, anti-CD4, anti-CD8, B220, and DX5 were purified from spleen cells of both naive and 15-12RM-injected mice at day 3 after 15-12RM injection, and TGF-β1 production *ex vivo* was examined without *in vitro* stimulation.

### Methods

Non-T non-B non-NK cells, referred to as non-lymphoid cells, were purified from spleen cells of BALB/c mice 3 days after 15-12RM injection. Spleen cells were depleted of T cells, B cells, and NK cells using magnetic beads coated with anti-Thy 1.2, anti-CD4, anti-CD8, anti-B220, and anti-DX5 (Miltenyi Biotec; Auburn, CA). The cells were cultured at a density of 2 x 10⁵/well of a 96 well plate in 200 µl of X Vivo 20 medium (BioWhittaker, Inc., Walkersville, MD). At the indicated times, 100 µl of culture medium was harvested from each well and stored at -80°C until cytokine measurement.

After acidification of the samples, to convert all TGF-β1 to the active form, the concentration of total TGF-β1 in the culture supernatant was determined by ELISA kit (R&D) according to the manufacturer's instructions. All samples were assayed in triplicate and each value is the average ± standard deviation (SD) of the triplicate assay. *p*<0.005 at 6 hours and *p*<0.01 at 12 hours with Student's t-test between naive and tumor bearing groups. Without acidification, no TGF-β1 was detected, indicates that it was all in the latent form The amount of TGF-β1 production *ex vivo* by 1 x 10⁶ cells was calculated as follows: concentration of culture supernatant (pg/ml) x 0.2 (ml)/(2 x 10⁵/1 x 10⁶) = pg produced per million cells. The data were analyzed for statistical significance using a Log-Rank test or Student's t-test. The data were considered significant at p<0.05.

### Results

Latent TGF-β1, although not detected at 3 hours of culture, was already detected in the culture supernatants of the non-lymphoid cells from 15-12RM-injected mice after only 6 hours of culture, but was undetected in cells from naive mice at that time point (p<0.005 by Student's t-test) (FIG. 4A). This earliest time point probably reflects the *in vivo* activity of these cells at the time they were removed from the mouse, without any *in vitro* stimulation. By 12 hours in tissue culture plastic, even the cells from non-tumor-bearing mice were making some TGF-β, suggesting a non-specific stimulation by the tissue culture plates or medium. Nevertheless, even at this later time point, the cells from 15-12RM-injected mice still produced more TGF-β1 than the cells from naive mice (p<0.01 by Student's t-test). The kinetics of TGF-β1 production by non-lymphoid cells was also examined (FIG. 4B). The TGF-β1 production by non-lymphoid cells was increased until at least day 10 after tumor injection.

### EXAMPLE 5

### TGF-β1 is Necessary for Tumor Recurrence In Vivo

To test the hypothesis that TGF-β1 was necessary for tumor recurrence *in vivo,* either an anti-CD4 antibody, a neutralizing anti-TGF-β monoclonal antibody (1D11.16; Genzyme, Cambridge, MA) or an isotype matched monoclonal antibody (13C4; Genzyme, Cambridge, MA) were injected intraperitoneally into mice that had received tumor cells.

### Methods

Since it was previously shown that the first ten days of 15-12RM injection were critical for negative regulation of tumor immunosurveillance (Terabe et al., Nature Immunol. 1:515, 2000), mice were treated with anti-CD4, anti-TGF-β, or the isotype matched monoclonal antibody for the first 10 days after tumor injection. The anti-CD4 monoclonal antibody (0.5 mg) was inoculated on day 0, 1, 2, 6, and 10 after tumor injection. 100 µg of anti-TGF-β monoclonal antibody or isotype matched control monoclonal antibody were injected every other day from day 0 to day 10.

In other experiments, after subcutaneous inoculation of 15-12RM cells, the mice (5/group) were treated with 100 µg of anti-TGF-β antibody from day 0 of day 5, or control isotype-matched antibody every other day for ten days. Size of primary tumors in 15-12RM-injected BALB/c mice treated with 100 µg of anti-TGF-β antibody intraperitonealy every other day for ten days, or without any antibody treatment, was measured in two perpendicular dimensions with calipers every other day.

### Results

The mice treated with anti-TGF-β monoclonal antibody were protected from the recurrence of tumor similarly to anti-CD4-treated (FIG. 4C) or IL-13 inhibitor-treated mice. The effect of anti-TGF-β was seen only in the recurrent phase when the difference in tumor size was enormous (>400 mm² in the controls versus none in the anti-TGF-β-treated mice). In contrast, there was little difference in the primary tumors. Although the sizes of the primary tumors during the initial growth period in anti-TGF-β-treated mice were slightly smaller than those of tumors in control mice in all the experiments, the difference was not significant (FIG. 4E). These results demonstrate that TGF-β1 is necessary for the tumor recurrence.

Even though increased TGF-β production was observed through day 10 (FIG. 4B), starting the anti-TGF-β-treatment at 5 day was too late to make an impact on late tumor recurrence (FIG. 4D). These results suggest that TGF-β1 produced early after tumor injection plays a critical role in down-regulation of anti-tumor immunosurveillance. The fact that blockade of TGF-β for the first 10 days is sufficient suggests that it is critical only during the priming of CTL, consistent with the observation that TGF-β inhibits the induction of CTL (FIG. 1B) but not their effector function.

### EXAMPLE 6

### Down-Regulation of In Vivo TGF-β1 Production

Although TGF-β1 might be induced by IL-13 and act downstream to down-regulate CTL mediated tumor immunosurveillance, the possibility remained that both IL-13 and TGF-β1 were necessary, and acting in parallel, to inhibit tumor immunosurveillance, albeit on different cell populations. To distinguish between these possibilities, the effect of an IL-13 inhibitor, which protects mice from tumor recurrence, on TGF-β1 production in tumor-bearing mice three days after tumor inoculation was tested.

### Methods

To measure the effect of an IL-13 inhibitor on TGF-β production, splenic non-lymphoid cells were prepared from naive and 15-12RM-injected mice with or without IL-13 inhibitor treatment intraperitonealy on days 0, 1, 2 following tumor injection. Two hundred thousand cells were then cultured *in vitro* without any stimulation, as in FIG 4A, above.

It has been shown that CD1-restricted NKT cells were responsible for IL-13 production induced in tumor-bearing mice and CD1 KO mice that lack NKT cells were resistant against tumor recurrence. Thus, the TGF-β1 production in two hundred thousand splenic non-lymphoid cells from wild-type BALB/c mice (naïve), 15-12RM tumor-injected BALB/c mice, or tumor-injected CD1 KO mice at day 3 post-injection was also examined. CD1 KO mice with BALB/c background were bred, and maintained, under pathogen-free conditions and were used at 6-10 weeks of age.

### Results

As shown in FIG. 5A, the cells from 15-12RM-injected mice produced more TGF-β1 than the cells from naive mice, consistent with the results shown in FIG. 4A (Example 4, above). However, the IL-13 inhibitor treatment reduced the TGF-β1 level to almost the same level seen in naive mice.

As shown in FIG. 5B, the cells from CD1 KO mice failed to produce TGF-β1 immediately *ex vivo,* in contrast to the cells from wild-type BALB/c mice. These results showed that in the tumor model, TGF-β1 expression by non-lymphoid cells in tumor-bearing animals is dependent *in vivo* on IL-13 and CD1d-restricted T cells.

### EXAMPLE 7

### Up-Regulation of TGF-β1 Production in CD11b⁺Gr-1⁺ Cells from Tumor-Bearing Mice

This Example shows that TGF-β1 is up-regulated by CD11b⁺Gr-1⁺ cells from tumor-bearing mice.

### Methods

Non-lymphoid spleen cells were obtained from naive and 15-12RM-injected mice (day 3) by depleteing CD4⁺, CD8⁺, B220⁺, and DX5⁺ cells with magnetic beads. The cells were stained with anti-Gr-1, anti-CD11c, anti-F4/80 and anti-CD11b antibodies for 30 minutes after blocking CD16/CD32 (anti-FcR 2.4G2, Pharmingen, San Diego, CA) for 15 minutes. The cells were washed once and fixed with Cytofix/Cytoperm (Pharmingen), and washed with Perm/Wash buffer. After washing, the cells were analyzed by FACScan or FACS Caliber by using CELLQuest software (BD Biosciences).

To determine which cell population in splenic non-lymphoid cells makes TGF-β1, on day three after 15-12RM injection, non-lymphoid spleen cells were purified from naive BALB/c (white bar) and 15-12RM tumor-injected BALB/c mice (black bars). CD11b⁺ and CD11c⁺ cells were depleted during the purification of the splenic non-lymphoid cells. For Gr-1 cell-depletion, mice were injected intraperitonealy with 200, 100, 10, or 1 µg of anti-Gr-1 antibody (Cederlane Laboratories Ltd., Ontario, Canada) on days 5, 6, 10,15, 20 after tumor injection. Two hundred thousand purified cells were cultured *in vitro* without particular stimulation. The culture supernatant was collected after 6 or 12 hours in culture and the concentration of TGF-β1 was determined by ELISA. Each value is the average ± SD of triplicate assays.

Intracellular cytokine staining was also performed to confirm TGF-β1 production by CD11b⁺Gr-1⁺ cells. Freshly isolated spleen cells from naïve and 15-12RM-injected mice were stained with anti-CD11b and anti-Gr-1. Then cells were fixed, permeabilized and stained with anti-TGF-β1 antibody.

### Results

In one specific experiment (FIG. 6), the splenic non-lymphoid spleen cells include CD11b⁺ (45.33 ± 10.23 % in naive mice, 33.72 ± 8.61 % in tumor-bearing mice), CD11c⁺ (3.78 ± 1.21 % in naive mice, 3.47 ± 1.76 % in tumor-bearing mice), F4/80⁺ (23.75 ± 9.80 % in naive mice, 17.83 ± 7.21 % in tumor-bearing mice) and Gr-1⁺ cells (45.09 ± 7.59 % in naïve mice, 35.92 ± 11.88 % in tumor-bearing mice). Among the CD11b⁺ cells, almost 80 % (37.52 ± 7.72 % and 28.16 ± 10.68 % of non-lymphoid spleen cells in naive and tumor-bearing mice) were positive for Gr-1 and more than 50 % (23.75 ±9.8 % and 17.83 ± 7.21 % % of non-lymphoid spleen cells in naive and tumor-bearing mice) were positive for F4/80. Most of the CD11c⁺ cells were myeloid dendritic cells that also expressed CD11b (FIG. 6).

Surprisingly, depletion of CD11b⁺ cells almost completely abrogated TGF-β1 production by non-lymphoid cells from tumor-bearing mice at 6 hours (FIG. 7A). Furthermore, when the Gr-1⁺ population was depleted, TGF-β1 production was drastically reduced at 6 hours (FIG. 7B). However, removing CD11c⁺ cells did not have an effect on the TGF-β1 level at 12 hours, although it did at six hours, whereas the effect of either CD11b or Gr-1 depletion persisted at 12 hours. This indicated that CD11c⁺ cells were less critical than CD11b⁺ Gr-1⁺ double positive cells for TGF-β production (FIGS. 7A and 7B). These results suggested that while CD11c⁺ cells may play some role, among the cells expressing CD11b, CD11b⁺Gr-1⁺ double positive cells were the major source of TGF-β1 production by non-lymphoid cells from 15-12RM injected mice. Anti-CD11b or anti-CD11c magnetic beads (Mylteni Biotech) were used at the same time with anti-Thy1.2, anit-B220, and anti-DX5 antibodies.

Based on surface staining against CD11b and Gr-1, there were at least two cell populations in double positive cells that could be distinguished by the level of Gr-1 expression. From anti-TGF-β1 staining in CD11b⁺Gr-1^{high} and CD11b⁺Gr-1^{low} cells, it appears that up-regulation of TGF-β1 production in spleen cells from 15-12RM-injected mice occurred in the CD11b⁺Gr-1^{high} but not the CD11b⁺Gr-1^{low} subpopulation.

The role of Gr-1⁺ cells in down-regulation of tumor immunosuveillance *in vivo* was examined. Mice were treated with various doses (200 µg, 100 µg, 10 µg, or 1 µg) of anti-Gr-1 (Cederlane Laboratories Ltd., Ontario, Canada). Among the doses, the group of mice treated with 1 µg of antibody between day 5 and day 20 following tumor injection were protected from tumor recurrence (FIG. 8C). Thus, Gr-1⁺ cells were not only necessary. for *ex vivo* TGF-β1 production, but in fact necessary for down-regulation of immunosurveillance *in vivo.*

### EXAMPLE 8

### Expression of Cell Surface Markers on Gr-1⁺CD11b⁺ Cells and Morphology of Gr-1⁺CD11b⁺ Cells

In order to better characterize Gr-1⁺CD11b⁺ cells, the expression of CD11c, CD31, and IL-4Ra on Gr-1⁺CD11b⁺ cells, as well as the morphology of these cells, were studied.

### Methods

On day 3 after 15-12RM injection, single-cell suspensions of spleen cells from tumor-bearing and naïve BALB/c mice were prepared for flow cytometry analysis. The cells were stained with FITC-conjugated anti-Gr-1, Per-CP conjugated anti-CD11b antibodies in combination with phycoerythrin (PE)-conjugated anti-F4/80, APC-conjugated anti-CD31, or PE-conjugated anti-IL-4Ra antibodies and analysed by flow cytometry. During the analysis of the data acquired, Gr-1⁺CD11b⁺ cells were gated and analysed for the expression of other molecules.

The morphology of Gr-1⁺CD11b⁺ cells was also examined. Non-lymphoid cells prepared from BALB/c spleen cells were stained with FITC-conjugated anti-Gr-1 and APC-conjugated anti-CD11b antibodies for 30 minutes after blocking CD16/CD32 (Pharmingen) for 15 minutes. After washing, the cells were gated on Gr-1^{hi}CD11b⁺ and Gr-1^{int}CD11b⁺ cell populations and sorted by FACSVantage (BD Bioscience). Sorted cells were collected by cytospin onto glass slide, and dried completely. The cells were stained with Wright-Giemsa stain using a Diff-Quik stain set (Dade Behring Inc., Deerfield, IL). Since there were two distinct cell populations among the Gr-1⁺CD11b⁺ cells based on the fluorescent intensity of Gr-1 staining, which is referred to as Gr-^{hi}CD11b⁺ and Gr-1^{int}CD11b⁺, each cell population was sorted and the morphology of the cells in each population was examined.

### Results

F4/80 was expressed on around 75% of the Gr-1⁺CD11b⁺ cells from both naïve and 15-12RM-injected mice. CD31 was expressed relatively higher on the cells from tumor-bearing mice compared with the cells from naïve mice (averages among several experiments were 24% and 19 % in tumor-bearing and naïve mice respectively), although the difference was not significant IL-4Rα, a component of the IL-13 receptor, was also expressed on the Gr-1⁺CD11b⁺ cells almost at the same level on naïve and tumor-bearing mice-derived cells (15-20%). Expression of IL-4Rα on the Gr-1⁺CD11b⁺ cells suggested that the Gr-1⁺CD11b⁺ cells express IL-13 receptor. This result is consistent with the result in FIG. 5B that *in vivo* blockade of IL-13 reduced *ex vivo* TGF-β production by these cells.

Gr-1^{hi}CD11b⁺ cells from both naïve and 15-12RM-injected mice were primarily mature and some immature neutrophils, and no difference was observed between the cells from naïve and 15-12RM-injected mice (FIG. 8B, upper two panels). Gr-1^{int}CD11b⁺cells (FIG. 8B, lower two panels) represented primarily immature myeloid cells, so-called "bands" (indicated by arrow heads), with some immature monocytes (indicated by arrows). The population from the 15-12RM-injected mice contained relatively fewer monocytes than the cells from the naive mice.

### EXAMPLE 9

### Role of Nitric Oxide (NO) in Suppression of T Cell Responses

Gr-1⁺CD11b⁺ myeloid cells induced by tumors may suppress T cell responses via nitric oxide. Thus, a possible role for NO tumor suppression *in vivo* was investigated in this Example.

Mice received 0.2 mg of L-NAME (*N*-nitro-L-arginine-methyl ester), which is believed to inhibit iNOS (inducible nitric oxide synthase) *in vivo,* or D-NAME (*N*-nitro-D-arginine-methyl ester) every day for two weeks after tumor injection. L-NAME treatment did not alter tumor growth *in vivo* (FIG. 9). Therefore, in 15-12RM injected mice, NO is not necessary for negative regulation of CD8⁺ CTL.

### EXAMPLE 10

### The Effect of anti-TGF-β on Metastasis In Vivo

The effect of the anti-TGF-β monoclonal antibody on tumor growth *in vivo,* using a mouse lung metastasis model of syngeneic colon carcinoma, is shown in this Example.

### Methods

Mouse colon carcinoma line CT26 was maintained in CTM. Twenty thousand CT26 cells were injected via the tail vein into BALB/c mice. The mice were inoculated intraperitonealy with 0.1 mg of either anti-TGF-β monoclonal antibody (1D11.16; Genzyme, Cambridge, MA) or control monoclonal antibody (13C4; Genzyme, Cambridge, MA) every other day for two weeks. At the time when control mice had developed a sufficient number of tumor metastases (around 3 weeks after tumor inoculation), all the mice were sacrificed and the lungs were perfused with a 15% solution of India ink. After fixation by Fekete's solution, lungs were evaluated for pulmonary metastases by macroscopically counting nodules in the lungs.

### Results

The maximum number of the nodules counted per lung was 250. Anti-TGF-β treatment significantly reduced the number of lung metastases. This result indicates that the role of TGF-β in down-regulation of CTL-mediated tumor immunosurveillance is not unique to the recurrence of a 15-12RM fibrosarcoma.

## Claims

1. An antibody which is a monoclonal antibody obtained from hybridoma 1 D 11.16 (ATCC Accession No. HB 9849) or is a humanized version thereof for use in a method of inhibiting recurrence of a tumor in a subject, wherein the monoclonal antibody is specific for TGF-β and neutralizes an activity of TGF-β, thereby inhibiting recurrence of the tumor in the subject.

2. Use of an antibody which is a monoclonal antibody obtained from hybridoma 1D11.16 (ATCC Accession No. HB 9849) or is a humanized version thereof in the manufacture of a medicament for inhibiting recurrence of a tumor in a subject, wherein the monoclonal antibody is specific for TGF-β and neutralizes an activity of TGF-β, thereby inhibiting recurrence of the tumor in the subject.

3. The antibody for use according to claim 1 or the use according to claim 2, wherein the monoclonal antibody blocks an immunosuppressive effect of transforming growth factor (TGF)-β in the subject.

4. The antibody for use or the use according to claim 3, wherein the monoclonal antibody inhibits TGF-β from binding a TGF-β receptor.

5. The antibody for use according to claim 1 or the use according to claim 2, wherein the subject is a human.

6. The antibody for use according to claim 1 or the use according to claim 2, wherein the tumor is benign or malignant.

7. The antibody for use according to claim 1 or the use according to claim 2, wherein the tumor comprises a carcinoma, a sarcoma, a leukemia, a lymphoma, or a tumor of the nervous system.

8. The antibody for use according to claim 1 or the use according to claim 2, wherein the tumor comprises a breast tumor, a liver tumor, a pancreatic tumor, a gastrointestinal tumor, a colon tumor,a uterine tumor, an ovarian tumor, a cervical tumor, a testicular tumor, a brain tumor, a skin tumor, a melanoma, a retinal tumor, a lung tumor, a kidney tumor, a bone tumor, a prostate tumor, a nasopharygeal tumor, a thryoid tumor, a leukemia, or a lymphoma.

9. The antibody for use according to claim 1 or the use according to claim 2, wherein the antibody is for administration intravenously, subcutaneously, intradermally, or intramuscularly.

10. The antibody for use according to claim 1 or the use according to claim 2, wherein the monoclonal antibody results in a lack of tumor growth *in vivo* or *in vitro*

11. The antibody for use according to claim 1 or the use according to claim 2, wherein in the method blocking the immunosuppressive effect of the TGF-β results in increased immunosurveillance by lymphocytes of the subject.

12. The antibody for use or the use according to claim 11, wherein the lymphocytes comprise T cells or B cells.

13. The antibody for use or the use according to claim 11, wherein the lymphocytes include T cells, and the T cells comprise a cytotoxic T lymphocyte (CTL), a CD8⁺ CTL, a CD4⁺ cell, a CD4⁺ CDld-restricted T cell, an NKT cell, or a combination thereof.

14. The antibody for use or the use according to claim 11, wherein increased immunosurveillance is measured by an increased biological activity of the lymphocyte.

15. The antibody for use or the use according to claim 14, wherein the increased activity of the lymphocyte is measured by a CTL assay.

16. The antibody for use or the use according to claim 15, wherein the CTL assay comprises a chromium release assay.

17. The antibody for use according to claim 1 or the use according to claim 2, wherein the monoclonal antibody inhibits TGF-β receptor signaling.

18. The antibody for use according to claim 1 or the use according to claim 2, wherein the monoclonal antibody blocks a TGF-β signaling pathway on contact with a TGF-β receptor-expressing immune cell and wherein blocking the TGF-β signaling pathway results in increased tumor immunosurveillance by the TGF-β receptor-expressing immune cell, thereby enhancing the activity of the immune cell to inhibit recurrence of the tumor.

19. The antibody for use or the use according to claim 18, wherein the TGF-β receptor-expressing immune cell is a T cell or a B cell.

20. The antibody for use or the use according to claim 18, wherein the TGF-β receptor-expressing immune cell includes T cells and the T cells comprise a CTL, a CD8⁺ CTL, a CD4⁺ cell, a CD4⁺ CD1d-restricted T cell, or an NKT cell.

## Patentansprüche

1. Antikörper, der ein von Hybridom 1 D11.16 (ATCC-Zugangsnummer HB 9849) erhaltener monoklonaler Antikörper oder eine humanisierte Version davon ist, zur Verwendung in einem Verfahren zur Hemmung des Wiederauftretens eines Tumors in einem Individuum, wobei der monoklonale Antikörper für TGF-β spezifisch ist und eine Aktivität von TGF-β neutralisiert, wodurch das Wiederauftreten des Tumors in dem Individuum gehemmt wird.

2. Verwendung eines Antikörpers, der ein von Hybridom 1D11.16 (ATCC-Zugangsnummer HB 9849) erhaltener monoklonaler Antikörper oder eine humanisierte Version davon ist, zur Herstellung eines Medikaments zur Hemmung des Wiederauftretens eines Tumors in einem Individuum, wobei der monoklonale Antikörper für TGF-β spezifisch ist und eine Aktivität von TGF-β neutralisiert, wodurch das Wiederauftreten des Tumors im Individuum gehemmt wird.

3. Antikörper zur Verwendung nach Anspruch 1 oder Verwendung nach Anspruch 2, wobei der monoklonale Antikörper eine immunsuppressive Wirkung eines transformierenden Wachstumsfaktors β (TGF-β) im Individuum blockiert.

4. Antikörper zur Verwendung oder Verwendung nach Anspruch 3, wobei der monoklonale Antikörper TGF-β daran hindert, einen TGF-β-Rezeptor zu binden.

5. Antikörper zur Verwendung nach Anspruch 1 oder Verwendung nach Anspruch 2, wobei das Individuum ein Mensch ist.

6. Antikörper zur Verwendung nach Anspruch 1 oder Verwendung nach Anspruch 2, wobei der Tumor gutartig oder bösartig ist.

7. Antikörper zur Verwendung nach Anspruch 1 oder Verwendung nach Anspruch 2, wobei der Tumor ein Karzinom, ein Sarkom, Leukämie, ein Lymphom oder einen Tumor des Nervensystems umfasst.

8. Antikörper zur Verwendung nach Anspruch 1 oder Verwendung nach Anspruch 2, wobei der Tumor einen Brusttumor, einen Lebertumor, einen Pankreastumor, einen gastrointestinalen Tumor, einen Kolontumor, einen Uterustumor, einen Ovarialtumor, einen Zervixtumor, einen Hodentumor, einen Hirntumor, einen Hauttumor, ein Melanom, einen Retinatumor, einen Lungentumor, einen Nierentumor, einen Knochentumor, einen Prostatatumor, einen Nasopharynxtumor, einen Schilddrüsentumor, Leukämie oder ein Lymphom umfasst.

9. Antikörper zur Verwendung nach Anspruch 1 oder Verwendung nach Anspruch 2, wobei der Antikörper zur intravenösen, subkutanen, intradermalen oder intramuskulären Verabreichung dient.

10. Antikörper zur Verwendung nach Anspruch 1 oder Verwendung nach Anspruch 2, wobei der monoklonale Antikörper in vivo oder in vitro zu fehlendem Tumorwachstum führt.

11. Antikörper zur Verwendung nach Anspruch 1 oder Verwendung nach Anspruch 2, wobei das Verfahren zum Blockieren der immunsuppressiven Wirkung des TGF-β zu einer erhöhten Immunüberwachung durch Lymphozyten des Individuums führt.

12. Antikörper zur Verwendung oder Verwendung nach Anspruch 11, wobei die Lymphozyten T-Zellen oder B-Zellen umfassen.

13. Antikörper zur Verwendung oder Verwendung nach Anspruch 11, wobei die Lymphozyten T-Zellen umfassen und die T-Zellen einen zytotoxischen T-Lymphozyten (CTL), einen CD8⁺-CTL, eine CD4⁺-Zelle, eine CD4⁺-CD1d-restringierte T-Zelle, eine NKT-Zelle oder eine Kombination davon umfassen.

14. Antikörper zur Verwendung oder Verwendung nach Anspruch 11, wobei eine erhöhte Immunüberwachung mittels einer erhöhten biologischen Aktivität des Lymphozyten gemessen wird.

15. Antikörper zur Verwendung oder Verwendung nach Anspruch 14, wobei die erhöhte Aktivität des Lymphozyten mithilfe eines CTL-Tests gemessen wird.

16. Antikörper zur Verwendung oder Verwendung nach Anspruch 15, wobei der CTL-Test einen Chromfreisetzungstest umfasst.

17. Antikörper zur Verwendung nach Anspruch 1 oder Verwendung nach Anspruch 2, wobei der monoklonale Antikörper TGF-β-Rezeptor-Signalübertragung hemmt.

18. Antikörper zur Verwendung nach Anspruch 1 oder Verwendung nach Anspruch 2, wobei der monoklonale Antikörper bei Kontakt mit einem TGF-β-Rezeptoren exprimierenden Immunozyten einen TGF-β-Signalübertragungsweg blockiert und wobei das Blockieren des TGF-β-Signalübertragungswegs zu erhöhter Tumor-Immunüberwachung durch den TGF-β-Rezeptoren exprimierenden Immunozyten führt, wodurch die Aktivität des Immunozyten zur Hemmung des Wiederauftretens des Tumors erhöht wird.

19. Antikörper zur Verwendung oder Verwendung nach Anspruch 18, wobei der TGF-β-Rezeptoren exprimierende Immunozyt eine T-Zelle oder eine B-Zelle ist.

20. Antikörper zur Verwendung oder Verwendung nach Anspruch 18, wobei der TGF-β-Rezeptoren exprimierende Immunozyt T-Zellen umfasst und die T-Zellen einen CTL, einen CD8⁺-CTL, eine CD4⁺-Zelle, eine CD4⁺-CD1d-restringierte T-Zelle oder eine NKT-Zelle umfassen.

## Revendications

1. Anticorps qui est un anticorps monoclonal obtenu de l'hybridome 1D11.16 (ATCC No. d'Accès HB 9849) ou est une version humanisée de celui-ci pour utilisation dans une méthode d'inhibition de la récurrence d'une tumeur dans un sujet, où l'anticorps monoclonal est spécifique à TGF-β et neutralise une activité de TGF-β en inhibant ainsi la récurrence de la tumeur dans le sujet.

2. Utilisation d'un anticorps qui est un anticorps monoclonal obtenu de l'hybridome 1D11.16 (ATCC No. d'Accès HB 9849) ou est une version humanisée de celui-ci dans la fabrication d'un médicament pour inhiber la récurrence d'une tumeur dans un sujet, où l'anticorps monoclonal est spécifique à TGF-β et neutralise une activité de TGF-β, en inhibant ainsi la récurrence de la tumeur dans le sujet.

3. Anticorps pour utilisation selon la revendication 1 ou l'utilisation selon la revendication 2, où l'anticorps monoclonal bloque un effet immunosuppressif du facteur de croissance transformant (TGF) -β dans le sujet.

4. Anticorps pour utilisation ou l'utilisation selon la revendication 3, où l'anticorps monoclonal inhibe la liaison de TGF-β à un récepteur de TGF-β.

5. Anticorps pour utilisation selon la revendication 1 ou l'utilisation selon la revendication 2, où le sujet est humain.

6. Anticorps pour utilisation selon la revendication 1, ou l'utilisation selon la revendication 2, où la tumeur est bénigne ou maligne.

7. Anticorps pour utilisation selon la revendication 1 ou l'utilisation selon la revendication 2, où la tumeur comprend un carcinome, un sarcome, une leucémie, un lymphome ou une tumeur du système nerveux.

8. Anticorps pour utilisation selon la revendication 1, ou l'utilisation selon la revendication 2, où la tumeur comprend une tumeur du sein, une tumeur du foi, une tumeur du pancréas, une tumeur gastro-intestinale, une tumeur du côlon, une tumeur de l'utérus, une tumeur des ovaires, une tumeur cervicale, une tumeur testiculaire, une tumeur du cerveau, une tumeur de la peau, un mélanome, une tumeur rétinale, une tumeur des poumons, une tumeur des reins, une tumeur d'os, une tumeur de la prostate, une tumeur naso-pharyngienne, une tumeur de la thyroïde, une leucémie ou un lymphome.

9. Anticorps pour utilisation selon la revendication 1, ou l'utilisation selon la revendication 2, où l'anticorps est pour l'administration de manière intraveineuse, sous-cutanée, intradermique ou intramusculaire.

10. Anticorps pour utilisation selon la revendication 1, ou l'utilisation selon la revendication 2, où l'anticorps monoclonal se traduit par une absence de croissance de la tumeur *in vivo* ou *in vitro.*

11. Anticorps pour utilisation selon la revendication 1 ou l'utilisation selon la revendication 2, où dans la méthode, le blocage de l'effet immunosuppressif de TGF-β se traduit par une immunosurveillance accrue par les lymphocytes du sujet.

12. Anticorps pour utilisation ou l'utilisation selon la revendication 11, où les lymphocytes comprennent des cellules T ou des cellules B.

13. Anticorps pour utilisation ou l'utilisation selon la revendication 11, où les lymphocytes comprennent des cellules T, et les cellules T comprennent un lymphocyte T cytotoxique (CTL), un CD8⁺CTL, une cellule CD4⁺, une cellule T CD4⁺ CD1d-restreinte, une cellule NKT ou une combinaison de ceux-ci.

14. Anticorps pour utilisation ou l'utilisation selon la revendication 11, où l'immunosurveillance accrue est mesurée par une activité biologique accrue du lymphocyte.

15. Anticorps pour utilisation ou l'utilisation selon la revendication 14, où l'activité accrue du lymphocyte est mesurée par un essai CTL.

16. Anticorps pour utilisation ou l'utilisation selon la revendication 15, où l'essai CTL comprend un essai de libération de chrome.

17. Anticorps pour utilisation selon la revendication 1 ou l'utilisation selon la revendication 2, où l'anticorps monoclonal inhibe la signalisation du récepteur de TGF-β.

18. Anticorps pour utilisation selon la revendication 1 ou l'utilisation selon la revendication 2, où l'anticorps monoclonal bloque un chemin de signalisation de TGF-β lors du contact avec une cellule immune d'expression du récepteur de TGF-β, et où le blocage du chemin de signalisation de TGF-β se traduit par une immunosurveillance accrue de la tumeur par la cellule immune exprimant le récepteur de TGF-β en renforçant ainsi l'activité de la cellule immune à inhiber la récurrence de la tumeur.

19. Anticorps pour utilisation ou l'utilisation selon la revendication 18, où la cellule immune d'expression du récepteur de TGF-β est une cellule T ou une cellule B.

20. Anticorps pour utilisation ou l'utilisation selon la revendication 18, où la cellule immune d'expression du récepteur de TGF-β comprend des cellules T, et les cellules T comprennent un CTL, un CD8' CTL, une cellule CD4⁺, une cellule T CD4⁺ CD1d-restreinte ou bien une cellule NKT.
